# EUROPEAN PATENT SPECIFICATION

(11) **EP 4 276 170 B1**
(45) Date of publication and mention of the grant of the patent: **29.04.2026**
(21) Application number: 22173043.5
(22) Date of filing: 12.05.2022
(51) Int. Cl.: C12N 1/14, C12N 11/04, C12N 11/10, C12M 1/16, C12M 1/40

(54) **PSEUDO-SOLID STATE FERMENTATION OF FILAMENTOUS FUNGI**
FERMENTATION VON FILAMENTÖSEN PILZEN MIT PSEUDO-FESTEM ZUSTAND
FERMENTATION À L'ÉTAT PSEUDO-SOLIDE DE CHAMPIGNONS FILAMENTEUX

(43) Date of publication of application: 15.11.2023
(73) Proprietor: Evologic Technologies GmbH, 1230 Wien (AT)
(72) Inventor: RUSCHITZKA, Paul, 1180 Wien (AT); BRILLMANN, Markus, 1140 Wien (AT); REICHELT, Wieland, 1160 Wien (AT); HERWIG, Christoph, 1130 Wien (AT)
(74) Representative: Maiwald GmbH

(56) References cited:
- WO-A1-2019/067379
- WO-A1-2019/140093
- SINGH O. V.: "Modulated gluconic acid production from immobilized cells ofAspergillus niger ORS-4.410 utilizing grape must", JOURNAL OF CHEMICAL TECHNOLOGY AND BIOTECHNOLOGY, vol. 83, no. 6, 5 February 2008 (2008-02-05), Hoboken, USA, pages 780 - 787, XP055972388, ISSN: 0268-2575, DOI: 10.1002/jctb.1866
- FERRAREZI A. L. ET AL: "Production and characterization of lipases and immobilization of whole cell of the thermophilic Thermomucor indicae seudaticae N31 for transesterification reaction", JOURNAL OF MOLECULAR CATALYSIS B : ENZYMATIC, vol. 107, 9 June 2014 (2014-06-09), pages 106 - 113, XP029038670, ISSN: 1381-1177, DOI: 10.1016/J.MOLCATB.2014.05.012
- MASLOVA O. ET AL: "Production of various organic acids from different renewable sources by immobilized cells in the regimes of separate hydrolysis and fermentation (SHF) and simultaneous saccharification and fermentation (SFF)", BIORESOURCE TECHNOLOGY, vol. 272, 29 September 2018 (2018-09-29), AMSTERDAM, NL, pages 1 - 9, XP055972323, ISSN: 0960-8524, DOI: 10.1016/j.biortech.2018.09.143
- DOTSENKO A. S. ET AL: "Complex effect of lignocellulosic biomass pretreatment with 1-butyl-3-methylimidazolium chloride ionic liquid on various aspects of ethanol and fumaric acid production by immobilized cells within SSF", BIORESOURCE TECHNOLOGY, vol. 250, 23 November 2017 (2017-11-23), AMSTERDAM, NL, pages 429 - 438, XP055972349, ISSN: 0960-8524, DOI: 10.1016/j.biortech.2017.11.064

## Description

### FIELD OF THE INVENTION

The present invention relates to methods of aseptically producing filamentous fungi in a bioreactor by pseudo-solid state fermentation. The present invention further relates to methods of continuously aseptically producing filamentous fungi in a bioreactor by pseudo-solid state fermentation.

### BACKGROUND OF THE INVENTION

Filamentous fungi are widely used in industry to produce small-molecule metabolites. Examples include antibiotics like penicillin, cholesterol-lowering drugs like Lovastatin, and food ingredients like citric acid. Because of their biological niche as microbial scavengers, filamentous fungi are also uniquely adapted to produce and secrete proteins. In environments rich in biological polymers, such as forest floors, the fungi thrive by secreting enzymes that degrade the polymers to produce monomers that can be readily used as nutrients for growth. The natural ability of fungi to produce proteins has been widely exploited, particularly in the production of industrial enzymes. Besides the production of metabolites, the utilization of fungi as biological control agents in agriculture is consistently rising in importance. Such fungal products, most often applied either in the form of spores or mycelium, help to maintain healthy crops to maintain crop yields, while reducing chemical pesticides and contaminants. Aspergillus, Metarhizium, Beauveria, Trichoderma, Colletotrichum, Chondrilla, Culicinomyces, Hirsutella, Nomuraea, Paecilomyces, Tolypocladium and Verticillium with their wide range of action against fungi, invertebrates, bacteria, and weeds are among the most prominent fungal representatives (S, J., Singh, D.P., 2016. Fungi as Biocontrol Agents in Sustainable Agriculture.). However, for widespread adoption of these solutions large scale production is required to reduce costs of manufacturing. Another field of great interest is the production of edible fungal material, for example as replacement of meat (Kumar et al., 2017. Meat Analogues: Health Promising Sustainable Meat Substitutes)

Industrial production of filamentous fungi and substances produced therein relies on large scale fermentation in bioreactors.

Fermentation processes can generally be categorized into two genres, solid state fermentations (SSF) and submerse fermentations (SmF). SSF is widely used for the cultivation of filamentous fungi and refers to cultivation of organisms on solids without or with hardly any free liquid. SmF, on the other hand, is based on liquid growth media as a continuous phase for cultivation. Each of these genres has their own advantages and disadvantages (Webb, 2017, Journal of Applied Biotechnology & Bioengineering, 4(1)), which are summarized in the below table:

| Possible drawbacks for the production of filamentous fungi in relation to fermentation mode | |
|---|---|
| **SSF** | **SmF** |
| Requires complete substrate sterilisation | Not the natural habitat for filamentous fungi |
| Uniform inoculation requires large amounts of inoculation media | Shear stress due to stirring and aeration |
| Product recovery from solid particles is laboriuos and costly | Technologically more challenging |
| Variability in composition of natural solid substrates | |
| Uneven distribution of nutrients | |
| Hard to control process | |

Less than 1% of the about 1.5 million fungal species originate from marine habitats. Accordingly, SSF resembles the natural habitat of the vast majority of fungi much better then SmF (Hölker, Höfer and Lenz, 2004, Applied Microbiology and Biotechnology, 64(2), pp. 175-186). Filamentous fungi can grow at lower moisture levels (as low as 20%) compared to bacterial cultivation requiring a minimum of 70% moisture, further rendering them suitable for SSF (Krishna, 2005, Critical Reviews in Biotechnology, 25(1-2), pp. 1-30). SSF mainly supports the formation of spores, whereas SmF can be useful for the production of mycelium. Indeed, conidia are more efficiently produced by SSF than in liquid fermentation (Jaronski, 2014, Mass Production of Beneficial Organisms - Invertebrates and Entomopathogens, Chapter 11, pp. 357-413). Spores that were cultivated in SSF tend to be advantageous in terms of desiccation resistance or virulence if used as biopesticides in later applications (Deshpande, 1999, Critical Reviews in Microbiology, 25(3), pp. 229-243).

The most common fermentation setups are: Tray Fermenter, Packed Bed Reactor, Continuously and Discontinuous Rotating Drum Bioreactors, Intermittently Stirred Beds, Bioreactor with Continuous Mixing and Forced Aeration (Krishna, 2005, Critical Reviews in Biotechnology, 25(1-2), pp. 1-30). These types are generally categorized based on their mixing and aeration systems. In tray fermentation, the substrate is evenly distributed on various layers or trays in a certain height, commonly up to a maximum of 15 centimeters. Oxygen input and removal of heat is mainly done via aeration surrounding the bead layers. During harvest, the trays commonly have to be removed from the bioreactor or cultivation chamber, which presents contamination risks and is cumbersome (Gowthaman, Krishna and Moo-Young, 2001, Applied Mycology and Biotechnology. Elsevier, pp. 305-352). Although it is not common, tray fermentation can also be conducted with forced aeration (Manan and Webb, 2020, Bioresources and Bioprocessing, 7(1), p. 16).

By-products of the agro-industry are often used as solid components in SSF processes. They mainly consist of starch, cellulose, lignocellulose, pectin, or other polysaccharides, and most often describe a very heterogeneous mixture (Krishna, 2005, Critical Reviews in Biotechnology, 25(1-2), pp. 1-30). As far as end product formulation is concerned, these by-products often have to be removed after fermentation. This process can be labor intensive and poses a high risk for contaminations.

Two types of solid phases are commonly used. One type utilizes solid substrate(s) as support matrix and nutrient source(s) at once. These solid substrates are often agricultural by-products such as different barns, straws, or pulps (Barrios-Gonzalez J. et al., 2005, Malaysian Journal of Microbiology. doi: 10.21161/mjm.110501). The second type employs inert solid substances which are impregnated with liquid media. In this type, the liquid fermentation broth can be extracted by pressing, whereby the solid support may be comprised of sugarcane, bagasse, pith, or polyurethane (Barrios-Gonzalez J. et al., 2005, Malaysian Journal of Microbiology. doi: 10.21161/mjm.110501). Since both types utilize solid particles as substrate or support, which are difficult or impossible to sterilize, state of the art SSF utilizes large amounts of highly active inoculum (10% of expected final biomass), preferably in the form of spores to reduce the risk of an overgrowing contamination (Roussos et al., 1991, Biotechnology Techniques, 5(6), pp. 415-420; Lonsane et al., 1992, Process Biochemistry, 27(5), pp. 259-273; Webb, 2017, Journal of Applied Biotechnology & Bioengineering, 4(1)).

Hydrogels and biopolymers, such as, e.g., alginate, have been used for storage of microorganisms, including filamentous fungi. Alginate consists of anionic linear watersoluble polysaccharides. Alginate beads are produced by dripping a Na+ alginate solution into a CaCl₂ bath. They have been used in submerse fermentations, i.e. in SmF, (Richter et al., 1989, Acta Biotechnol. 9 (1989) 2, 123-129; WO 2019140093 A1) and as an immobilization matrix for enzymes and encapsulation matrix for organisms for storage (Strobel et al., 2018, Industrial Biotechnology, 14(3), pp. 138-147). It is also well known that microorganisms such as *Neozygites parvispora* can grow inside and out of alginate beads, for example on microscope slides (Grundschober, Tuor and Aebi, 1998, Systematic and Applied Microbiology, 21(3), pp. 461-469). WO 2019067379 A1 utilizes alginate beads for the encapsulation of *Trichoderma* as an inoculum, which is used for inoculating liquid cultivation (SmF) or solid state fermentation on a solid substrate (SSF), the latter of which could also be mixed with liquids. However, hydrogel beads containing encapsulated fungi have merely been used for storage and/or as inoculation material, but not as sole growth matrix and growth substrate within an otherwise aseptic process inside a bioreactor without having to use antibiotics. The process of in situ steam sterilization as it can be performed with common solid particles is not applicable with hydrogel beads (WO1999057239A2). Introducing and distributing large amounts of beads to a bioreactor in a fully aseptic way represents a major challenge.

In summary, while being better suited to filamentous fungi than SmF, SSF carries a high risk of contamination by other fungi, which poses a threat to product quality and reproducibility.. Since solid material functions as either a carbon and nutrient source and/or as solid support matrix in form of an inert substrate, product recovery from solid material is necessary at the end of SSF, which is difficult and presents yet another risk of contamination due to sterility issues.

Accordingly, there is a need for alternative methods for filamentous fungi fermentation of all scales that enable the aseptic automation of the above mentioned processes and do not require an aseptic environment.

### SUMMARY OF THE INVENTION

The inventors have developed a new type of fermentation process, where the solid particles of SSF are replaced with hydrogel beads as the sole growth substrate and growth matrix in a fully aseptic process without having to use cost-intensive antibiotics, thereby circumventing some of the drawbacks of SSF as well as unlocking some other highly beneficial effects. This new process is termed pseudo-solid state fermentation ("PSSF").

The advantages of PSSF are as follows:
1) Reduction of contamination risk due to enabling fully aseptic process conditions since hydrogels can be sterilized in their liquid form either by autoclaving or, in the case of alginate, even sterile filtration (Smidsrod and Skjakbrk, 1990, Trends in Biotechnology, 8, pp. 71-78). This advantage, along with the potential of using defined media composition of hydrogel beads, enables production with constant product quality as well as long term and continuous cultivation.
2) Hydrogel beads consist of at least 50% and up to 97% water, which means they can be dried to tiny residues under mild drying conditions that do not harm the affiliated organisms. Alternatively, they can be fully dissolved. These possibilities create great flexibility in application and further usage as, e.g.:
   a. direct inoculum of beneficial microorganisms for agriculture in liquid form;
   b. direct inoculum of beneficial microorganisms for agriculture in beads of different levels of residual moisture; or
   c. contamination free solid or liquid inoculum for further fermentation processes, which overcomes the requirement for high amounts of inoculants for SSF to outcompete contaminants. Accordingly, this removes a direct limitation on scalability of SSF processes, since multiple scales for preculture generation would be required otherwise.
3) Due to fermentation on/in hydrogel beads, the produced filamentous fungi do not need to additionally be enclosed in hydrogel beads for storage and/or application at the end of the fermentation process. The beneficial effect of hydrogel encapsulation on storage viability of filamentous fungi has been shown by Pitaktamrong et al., 2018, Engineering Journal, 22(6), pp. 65-79.
4) Fungal primary metabolites are essential for growth and propagation. Secondary metabolites have various roles. The following classes are known as secondary metabolites: polyketides, terpenes, indole alkaloids, nonribosomal peptides. Secondary metabolites can function as antibiotic, antitumor, antioxidant, immunosuppressive and immune-stimulative agents. Besides their prominence in medicine, they play important direct and indirect roles in crop protection by reducing abiotic and biotic stresses on plants (Dashora et al., 2021, New and Future Developments in Microbial Biotechnology and Bioengineering. Elsevier, pp. 39-46). These valuable secondary metabolites are mainly produced under oxygen limited conditions within the hydrogel beads, where they can either protect the fungi during formulation or can be recovered via dissolving the hydrogel beads.
5) The importance of initial moisture content as well as active water during fermentation was reported by Barrios-Gonzalez J. et al., 2005, Malaysian Journal of Microbiology. doi: 10.21161/mjm.110501. Active water describes the amount of water that is not bound to the substrate and is defined as the partial vapor pressure of water in the solution divided by the partial vapor pressure of water at a defined temperature. The utilization of defined hydrogel beads containing at least 50% and up to 97% of water, paired with temperature control and humidified aeration, causes moisture content, as well as swelling and shrinking of hydrogel beads, to be controllable factors that allow for triggering metabolic events such as production of secondary metabolites or sporulation, at desired time points.

The object of the present invention is solved by the subject matter of the independent claims. Preferred embodiments are apparent from the dependent claims.

Accordingly, in one embodiment the present invention provides a method of aseptically producing filamentous fungi in a bioreactor by pseudo-solid state fermentation, comprising the steps of:
(a) introduction of hydrogel beads into a chamber (1) of the bioreactor in solution via an inoculation port (2) or
   in situ production of hydrogel beads inside the chamber (1) of the bioreactor by introduction of at least a crosslinking agent and a biopolymer solution into the chamber (1) via an inoculation port (2), optionally wherein the biopolymer solution comprises nutrients, followed by washing of the produced beads;
(b) if no nutrients are comprised by the biopolymer solution in step (a), equilibration of the hydrogel beads with a growth medium comprising nutrients;
(c) inoculation of filamentous fungi into the chamber (1) via the inoculation port (2);
(d) even distribution of the inoculated hydrogel beads on at least one perforated plate (3) arranged horizontally within the chamber (1) by means of aeration or stirring;
(e) removal of all or virtually all liquids from the chamber (1) via a harvest port (4);
(f) fermentation of the filamentous fungi inside the bioreactor in the absence of any or virtually any liquids to increase filamentous fungi biomass, wherein the hydrogel beads are the only growth substrate and growth matrix inside the bioreactor;
(g) optionally at least one introduction of growth medium followed by repetition of steps (e)-(f) to re-equilibrate the hydrogel beads;
(h) introduction of liquid to resuspend the hydrogel beads and filamentous fungi biomass and tearing the filamentous fungi biomass by aeration or stirring;
(i) harvest of the filamentous fungi biomass and hydrogel beads via the harvest port (4) by flushing;
(j) optional drying of the harvested material.

In a second embodiment, the present invention provides a method of aseptically producing filamentous fungi in a bioreactor by pseudo-solid state fermentation, comprising the steps of:
(a) introduction of hydrogel beads comprising viable filamentous fungi into a chamber (1) of the bioreactor in solution via an inoculation port (2) or
   in situ production of hydrogel beads inside the chamber (1) of the bioreactor by introduction of at least filamentous fungi, a crosslinking agent, and a biopolymer solution into the chamber (1) via an inoculation port (2), optionally wherein the biopolymer solution comprises nutrients, followed by washing of the produced beads;
(b) if no nutrients are comprised by the biopolymer solution in step (a), equilibration of the hydrogel beads with a growth medium comprising nutrients;
(c) even distribution of the hydrogel beads on at least one perforated plate (3) arranged horizontally within the chamber (1) by means of aeration or stirring;
(d) removal of all or virtually all liquids from the chamber (1) via a harvest port (4);
(e) fermentation of the filamentous fungi inside the bioreactor in the absence of any or virtually any liquids to increase filamentous fungi biomass, wherein the hydrogel beads are the only growth substrate and growth matrix inside the bioreactor;
(f) optionally at least one introduction of growth medium followed by repetition of steps (d)-(e) to re-equilibrate the hydrogel beads;
(g) introduction of liquid to resuspend the hydrogel beads and filamentous fungi biomass and tearing the filamentous fungi biomass by aeration or stirring;
(h) harvest of the filamentous fungi biomass and hydrogel beads via the harvest port (4) by flushing;
(i) optional drying of the harvested material.

In a third embodiment, the present invention provides a method of continuously, aseptically producing filamentous fungi in a bioreactor by pseudo-solid state fermentation, comprising the steps of:
(a) Introduction of hydrogel beads into a chamber (1) of the bioreactor in solution via an inoculation port (2) or
   in situ production of hydrogel beads inside the chamber (1) of the bioreactor by introduction of at least a crosslinking agent and a biopolymer solution into the chamber (1) via an inoculation port (2), optionally wherein the biopolymer solution comprises nutrients, followed by washing of the produced beads;
(b) if no nutrients are comprised by the biopolymer solution in step (a), equilibration of the inoculated hydrogel beads with a growth medium comprising nutrients;
(c) inoculation of filamentous fungi into the chamber (1) via the inoculation port (2);
(d) even distribution of the inoculated hydrogel beads on at least one perforated plate (3) arranged horizontally within the chamber (1) by means of aeration or stirring;
(e) removal of all or virtually all liquids from the chamber (1) via a harvest port (4);
(f) fermentation of the filamentous fungi inside the bioreactor in the absence of any or virtually any liquids to increase filamentous fungi biomass, wherein the hydrogel beads are the only growth substrate and growth matrix inside the bioreactor;
(g) optionally at least one introduction of growth medium followed by repetition of steps (e)-(f) to re-equilibrate the hydrogel beads;
(h) introduction of liquid to resuspend the hydrogel beads and filamentous fungi biomass and tearing the filamentous fungi biomass by aeration or stirring;
(i) draining liquid and suspended filamentous fungi biomass via the harvest port (4), wherein the harvest port comprises or is covered by a further perforated plate, wherein the openings in the further perforated plate have a smaller diameter than the hydrogel beads such that the hydrogel beads are retained in the chamber (1);
(j) equilibration of the hydrogel beads with a growth medium;
(k) even distribution of the hydrogel beads and any remaining filamentous fungi biomass on the at least one perforated plate (3) arranged horizontally within the chamber (1) by means of aeration or stirring;
(l) continuous repetition of steps (e)-(k);
(m) optional drying of the harvested material.

In a fourth embodiment, the present invention provides a method of aseptically producing filamentous fungi in a bioreactor by pseudo-solid state fermentation, comprising the steps of:
(a) Introduction of hydrogel beads comprising viable filamentous fungi into a chamber (1) of the bioreactor in solution via an inoculation port (2) or
   in situ production of hydrogel beads inside the chamber (1) of the bioreactor by introduction of at least filamentous fungi, a crosslinking agent, and a biopolymer solution into the chamber (1) via an inoculation port (2), optionally wherein the biopolymer solution comprises nutrients, followed by washing of the produced beads;
(b) if no nutrients are comprised by the biopolymer solution in step (a), equilibration of the hydrogel beads with a growth medium comprising nutrients;
(c) even distribution of the hydrogel beads on at least one perforated plate (3) arranged horizontally within the chamber (1) by means of aeration;
(d) removal of all or virtually all liquids from the chamber (1) via a harvest port (4);
(e) fermentation of the filamentous fungi inside the bioreactor in the absence of any or virtually any liquids to increase filamentous fungi biomass, wherein the hydrogel beads are the only growth substrate and growth matrix inside the bioreactor;
(f) optionally at least one introduction of growth medium followed by repetition of steps (d)-(e) to re-equilibrate the hydrogel beads;
(g) introduction of liquid to resuspend the hydrogel beads and filamentous fungi biomass and tearing the filamentous fungi biomass by aeration or stirring;
(h) draining liquid and suspended filamentous fungi biomass via the harvest port (4), wherein the harvest port comprises or is covered by a further perforated plate, wherein the openings in the further perforated plate have a smaller diameter than the hydrogel beads such that the hydrogel beads are retained in the chamber (1);
(i) equilibration of the hydrogel beads with a growth medium;
(j) even distribution of the inoculated hydrogel beads and any remaining filamentous fungi biomass on the at least one perforated plate (3) arranged horizontally within the chamber (1) by means of aeration or stirring;
(k) continuous repetition of steps (e)-(j);
(l) optional drying of the harvested material.

In an embodiment, in any of the above methods of the invention, the hydrogel beads comprise at least one polymer and water; preferably wherein the at least one polymer is selected from the group consisting of: alginate, carrageenan, and gellan gum and/or preferably wherein the water content of the hydrogel beads is at least 50%. In one such embodiment, the hydrogel beads further comprise a filler compound, preferably wherein the filler compound is selected from the group consisting of: talc, corn starch, corn flour, betonite, kaolinite, quartz silica powder, peat, arabic gum.

In an embodiment, in any of the above methods of the invention, the hydrogel beads have a size of 0.1mm to 6mm, preferably of 0.5 to 5mm, most preferably of 2mm to 4mm.

In an embodiment, in any of the above methods of the invention, the hydrogel beads form a layer with a height of 1cm to 150cm, preferably of 2cm to 50cm, most preferably of 5cm to 20cm.

In an embodiment, in any of the above methods of the invention, the hydrogel beads comprise at least one carbon source, preferably wherein the at least one carbon source is selected from the group consisting of: a sugar, and a starch; preferably wherein the sugar is selected from the group consisting of: monosaccharides and disaccharides, and wherein the starch is selected from the group consisting of: oligosaccharides and polysaccharides.

In an embodiment, in any of the above methods of the invention based on the first and third embodiment, inoculation with filamentous fungi is inoculation with spores.

In an embodiment, in any of the above methods of the invention, equilibration occurs for 1 to 28 hours, preferably 18 to 28 hours, most preferably 24 hours.

In an embodiment, in any of the above methods of the invention, during fermentation, the humidity inside the chamber (1) is controlled by aeration via a sparger (5), preferably wherein the humidity inside the chamber (1) is maintained at between 25% and 100%, preferably at between 70% and 100%.

In an embodiment, in any of the above methods of the invention, fermentation occurs for 6 hours to 120 days, preferably for 1 to 90 days.

In an embodiment, in any of the above methods of the invention, the filamentous fungi are selected from the list consisting of Zygomycota, Ascomycota, Basidiomycota and Glomeromycota. In a preferred embodiment, in any of the above methods of the invention, the filamentous fungi are selected from the list consisting of: *Ampelomyces, Aspergillus, Beauveria, Fusarium, Isaria, Lecanicillium, Metarhizium, Penicillium, Phoma, Purpureocillium, Serendipita, Trichoderma, Verticillium and* Mycorrhiza.

In an embodiment, in any of the above methods based on the first and second embodiments, the filamentous fungi are ectomycorrhiza or arbuscular mycorrhiza (AMF).

### BRIEF DESCRIPTION OF THE DRAWINGS

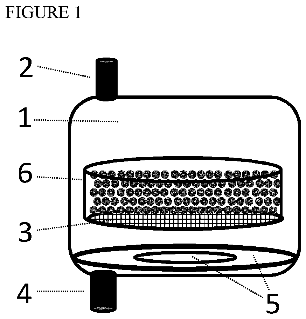

Figure 1 shows a bioreactor design for use in the inventive methods. The bioreactor comprises a chamber (1), an inoculation port (2), a perforated plate (3), a harvest port (4), a sparger (5) consisting of an outer ring sparger and an inner sparger, and a draft tube (6). Hydrogel beads are shown in their final positions as filled grey circles.

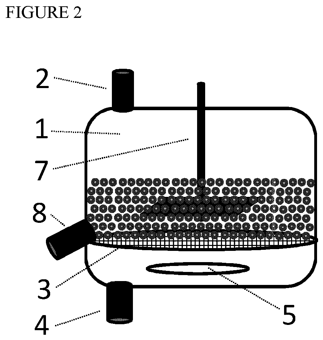

Figure 2 shows a bioreactor design for use in the inventive methods. The bioreactor comprises a chamber (1), an inoculation port (2), a perforated plate (3), a harvest port (4), a sparger (5), a stirrer (7), and a bead harvest port (8). Hydrogel beads are shown in their final positions as filled grey circles.

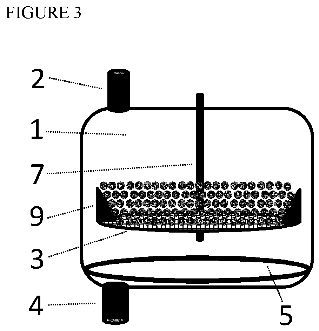

Figure 3 shows a bioreactor design for use in the inventive methods. The bioreactor comprises a chamber (1), an inoculation port (2), a perforated plate (3), a harvest port (4), a sparger (5), a stirrer (7), and a slope barrier (9). Hydrogel beads are shown in their final positions as filled grey circles.

### DETAILED DESCRIPTION OF THE INVENTION

The present invention, as illustratively described in the following, may suitably be practiced in the absence of any element or elements, limitation or limitations, not specifically disclosed herein.

The present invention will be described with respect to particular embodiments, but the invention is not limited thereto, but only by the claims.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group which preferably consists only of these embodiments.

Where an indefinite or definite article is used when referring to a singular noun, e.g. "a", "an" or "the", this includes a plural of that noun unless something else is specifically stated.

The term "aseptic environment" as used herein refers to conditions that prevent contamination with microorganisms by being free from microorganisms or having reduced numbers of microorganisms compared to the surrounding environment, with the maximal number of colony forming units per cubic meter (CFU/m³) being 200 (see EU GMP guidelines, clean room classification D). An aseptic environment can be, e.g., a clean room, a laminar flow bench, or a sterile space. An "aseptic" method or "aseptically" as used herein means that no interference with or removal from the bioreactor can introduce contaminants during the process. In the present invention, every component or material inside the bioreactor except the filamentous fungi inoculate has either been sterilized by steam sterilization in an autoclave or inside the bioreactor or via sterile filtration (0,2 µm).

The term "filamentous fungus" as used herein refers to fungi that form filamentous structures known as hyphae. These are multicellular structures with branching. Most of these hyphae extend in 3 dimensions through whatever they are growing in. Specialised hyphae are produced to allow vegetative (non-sexual) reproduction with spores or conidia. Some highly specialised reproductive or protective structures are also formed by some species, such as Ascospores. Filamentous fungi are found in most phylogenetic groups. Exemplary filamentous fungi are, e.g., from the phyla of Zygomycota, Ascomycota, Basidiomycota and Glomeromycota, e.g., *Ampelomyces, Aspergillus, Beauveria, Fusarium, Isaria, Lecanicillium, Metarhizium, Penicillium, Phoma, Purpureocillium, Serendipita, Trichoderma, Verticillium and* Mycorrhiza, such as arbuscular mycorrhizal fungi (AMF) and ectomyccorrhiza, both of which can also be cultivated in the absence of root material (Sugiura et al., 2020, PNAS, 117 (41) 25779-25788, which shows that myristate can be used as a carbon and energy source).

The term "filamentous fungi biomass" as used herein refers to any viable fungus, mycelium and/or spores.

The term "spore" refers to a unit of sexual or asexual reproduction that may be adapted for dispersal and for survival, often for extended periods of time, in unfavourable conditions. Spores form part of the life cycle of filamentous fungi. Spores are usually haploid and unicellular. Conidiospores or mitospores are produced asexually by mitosis; blastospores are produced via budding.

The terms "fermentation" or "fermenting" and "cultivation" or "cultivating" as used herein refer to the production of filamentous fungi by incubation at conditions suitable to elicit an increase in biomass and to ensure viability of the filamentous fungi. Necessary nutrients may be supplied to the filamentous fungi in a liquid growth medium, in a solid growth medium ("substrate"), or in hydrogel beads that comprise the nutrients or are equilibrated with nutrient medium. In pseudo-solid state fermentation, the nutrients are provided in the hydrogel beads as the substrate. The hydrogel beads may be formed by providing nutrients in a biopolymer that is mixed with a crosslinking solution, or the hydrogel beads may, after formation in the absence of nutrients, be equilibrated with a liquid growth medium comprising nutrients that is removed before fermentation. For equilibration, liquid growth medium can be applied to the hydrogel beads and/or filamentous fungi intermittently from above, e.g. by spraying or trickling, or the hydrogel beads and/or filamentous fungi can be intermittently submerged therein. Filamentous fungi fermentation according to the present invention occurs within bioreactors. Fermentation duration can span from 6 hours to longterm culture, e.g. up to 120 days or longer. In some embodiments, the fermentation occurs in a batch culture, a continuous culture, or a batch culture followed by fed batch culture.

"Continuous" aseptic fermentation as used herein refers to fermentation in which some filamentous fungi biomass is retained along with hydrogel beads inside the bioreactor during the aseptic harvest step, which then serves as the inoculum for the next fermentation cycle. That is, regular harvest occurs throughout the span of continuous fermentation, but no further filamentous fungi have to be added to the bioreactor.

The term "submerged fermentation" or "SmF" as used herein refers to culturing filamentous fungi by entirely submerging them in liquid growth medium throughout the duration of the fermentation.

The term "solid state fermentation" or "SSF" as used herein refers to culturing filamentous fungi on a solid substrate and/or matrix in the absence of any or virtually any liquid throughout the duration of the fermentation.

The term "pseudo-solid state fermentation" or "PSSF" as used herein refers to culturing filamentous fungi on hydrogel beads as the sole growth substrate and growth matrix externally added to the bioreactor in the absence of any or virtually any liquid, such as, e.g., liquid growth medium, nutrient solution, etc., throughout the duration of the fermentation.

The term "virtually any liquid" refers to 1% or less of liquid volume compared to bead volume.

The term "growth substrate" refers to a solid that provides nutrients for growth to filamentous fungi.

The term "growth matrix" refers to a solid that provides a surface on which filamentous fungi can grow.

The term "bioreactor" as used herein refers to a vessel suitable for fermenting filamentous fungi therein. A bioreactor suitable for the inventive methods will comprise at least a chamber (1) in which fermentation takes place, an inoculation port (2) through which inoculum and any other fermentation components such as, e.g., hydrogel beads, growth medium, etc., can be introduced into the chamber (1), at least one perforated plate (3) arranged horizontally within the chamber (1) on which hydrogel beads can come to rest, and a harvest port (4) through which produced filamentous fungi material and/or hydrogel beads and/or liquids can be removed from the chamber (1). The bioreactor will also further comprise at least a sparger (5), and may further comprise a stirrer (7).

The terms "port" and "ports" as used herein refer to an opening in a bioreactor, which can be fitted with a connector, a screw cap, or a rotary vacuum seal.

A suitable bioreactor, including all fittings, covers, ports, and outlets, is preferably made of metal, preferably of steel, most preferably of austentitic stainless steel, e.g. "WNr. 1.4404 (X2CrNiMo17-12-2), AISI 316L, (A4L)". Advantageously, such a bioreactor can be sterilized by autoclavation (either inside an autoclave or by internal application of steam at 121 degrees Celsius and 1 bar for at least 15 minutes) and has high resistance to corrosion due to chloride in washing or growth media containing chloride.

The chamber (1) is a chamber within the bioreactor in which the fermentation step is performed. The chamber (1) volume is from 1-100000 L, preferably from 10 - 100000L, more preferably from 1000-100000 L, most preferably from 10000-100000L.

The perforated plate (3) is preferably made of polypropylene or metal, preferably of steel, most preferably of austentitic stainless steel, e.g. "WNr. 1.4404 (X2CrNiMo17-12-2), AISI 316L, (A4L)". Advantageously, such a perforated plate (3) can be sterilized by autoclavation and has high resistance to corrosion due to chloride in washing or growth media containing chloride. The perforated plate (3) can be made from a perforated sheet of metal or from a metal grid.

The harvest port (4) is located at the bottom of the bioreactor, which permits the harvest of filamentous fungi material and/or hydrogel beads suspended in liquid by gravity. The harvest port (4) may comprise or be covered by a further perforated plate the openings of which have a smaller diameter than the hydrogel beads, such that the hydrogel beads cannot pass through the further perforated plate.

The term "sparger" as used herein refers to a device that bubbles sterilized air with a composition of 20-30 % O₂, 0.04-2 % CO₂, and 68-78.96% N₂ into the bioreactor. A sparger (5) can consist of one sparging element or multiple sparging elements. The sparger (5) may be any of the following types: sparging tube, sintered stainless steel sparger, porous stainless steel element, spider type sparger, ring sparger. A sparger can comprise an separately controllable inner sparger and an outer ring sparger (see e.g. Figure 1). The sparger (5) is preferably made of metal, more preferably of steel, most preferably of austentitic stainless steel, e.g. "WNr. 1.4404 (X2CrNiMo17-12-2), AISI 316L, (A4L)". Advantageously, such a sparger (5) can be sterilized by autoclavation and has high resistance to corrosion due to chloride in washing or growth media containing chloride.

Figure 1 shows an exemplary bioreactor design for use in the inventive methods. The bioreactor, in addition to the chamber (1), inoculation port (2), perforated plate (3), harvest port (4), and sparger (5), comprises a draft tube (6). The sparger (5) consists of an outer ring sparger with a diameter larger than the draft tube (6) and an inner sparger located beneath the draft tube (6). The outer sparger serves to create liquid circulation through the draft tube (6), which distributes the hydrogel beads into the draft tube to come to rest on the perforated plate (3) arranged inside the draft tube (6) in the distribution step of the inventive methods. This circulation can be reversed by means of the inner sparger in the tearing step of the inventive methods, such that the filamentous fungi biomass and hydrogel beads are pushed out of the draft tube and into those parts of the chamber (1) located outside the draft tube (6), and thus can be harvested through the harvest port (4).

Figure 2 shows an exemplary bioreactor design for use in the inventive methods. The bioreactor, in addition to the chamber (1), inoculation port (2), perforated plate (3), harvest port (4), and sparger (5), comprises a stirrer (7), and a bead harvest port (8). The stirrer distributes the hydrogel beads evenly throughout the liquid so that they settle in an even layer on the perforated plate (3), which spans the entirety of the chamber (1). The stirrer also stirs the resulting filamentous fungi biomass and hydrogel beads to tear the biomass. Liquid can be removed via the harvest port (4) located beneath the perforated plate (3) without removing the hydrogel beads. The hydrogel beads, either alone or together with filamentous fungi biomass, can be harvested via the bead harvest port (8) located above the perforated plate (3).

Figure 3 shows an exemplary bioreactor design for use in the inventive methods. The bioreactor, in addition to the chamber (1), inoculation port (2), harvest port (4), and sparger (5), comprises a stirrer shaft (7) where a perforated plate (3) with a slope barrier (9) is attached. The sparger (5) serves to create liquid circulation transporting the hydrogel beads onto the perforated plate (3) bordered by the slope barrier (9), arranged on the outer edge of the perforated plate (3). Rotation of the stirrer with the attached perforated plate (3) causes the filamentous fungi biomass and hydrogel beads to move towards the outer wall of the bioreactor and fall down to the bottom for harvest.

The term "hydrogel" as used herein refers to a network of crosslinked biopolymer chains that are hydrophilic, sometimes found as a colloid in which water is the dispersion medium. A three-dimensional solid results from the hydrophilic polymer chains being held together by cross-links. A "hydrogel bead" is a small, roughly spherical particle of hydrogel. Hydrogel beads are formed by admixing a biopolymer solution and a crosslinking agent, optionally wherein the biopolymer solution comprises nutrients, and dripping the resulting mixture to form beads. Hydrogel beads may be produced such that they contain viable filamentous fungi, e.g. spores of filamentous fungi, by admixing filamentous fungi, a biopolymer solution (optionally comprising nutrients), and a crosslinking agent. Hydrogel beads can be produced *in situ* inside the bioreactor of an inventive method in this manner. Exemplary biopolymers are alginate, carrageenan, and gellan gum. Hydrogels have a high water content, of at least 50%, preferably above 80%, e.g. 80%, 81%, 82%, 83%, 84%, 85%, 86%, 87%, 88%, 90%, 91%, 92%, 93%, 94%, 95%, 96% or 97% weight per weight. After production of hydrogel beads by admixing the biopolymer solution and crosslinking agent, the produced beads are washed to remove remaining crosslinking agent. "Washing" as used herein refers to addition and removal of a liquid, e.g. water, buffer, growth medium, or a saline solution. The term "buffer" as used herein is an aqueous solution comprising a mixture of a weak acid and its conjugate base, or vice versa. Buffer solutions are used as a means of keeping the pH at a nearly constant value in a wide variety of conditions and upon addition of a wide variety of substances.

A preferred hydrogel bead is an alginate bead. Alginate beads are produced by dripping a Na+ alginate solution (i.e. the biopolymer solution, optionally comprising nutrients) into a CaCl₂ (i.e. the crosslinking agent) bath.

Hydrogel beads may comprise at least one nitrogen source and one phosphor source, preferably wherein the phosphor source is in the form of PO₄³⁻. The nitrogen source preferably is available in the form of NO₂⁻, NO₃⁻, or NH4+, either from defined sources or complex sources such as e.g. yeast extract, peptone, potato infuse, corn steep liquor.

"Equilibration" or "equilibrating" of hydrogel beads as used herein refers to bringing hydrogel beads in contact with a liquid growth medium comprising nutrients for a period of time sufficient for nutrients to diffuse into the hydrogel beads from the growth medium. "Re-equilibrating" or "re-equilibration" of hydrogel beads as used herein means bringing hydrogel beads that previously contained sufficient amounts of nutrients, but no longer do so due to filamentous fungi using them up during fermentation., in contact with a liquid growth medium comprising nutrients for a period of time sufficient for nutrients to diffuse into the hydrogel beads from the growth medium.

"Nutrients" as used herein are substances used by an organism, e.g. a filamentous fungus, to survive, grow, and reproduce.

The terms "growth medium" and "nutrient medium" as used herein refers to a fermentation medium suitable to facilitate growth of filamentous fungi. The growth media of the present invention are typically a liquid growth medium containing nutrients that can diffuse into hydrogel beads. Suitable growth media for filamentous fungi are well known in the art. They can either be defined in terms of their exact chemical composition or can be complex. Exemplary complex growth media are Potato Dextrose Broth, Molasses Yeast Broth, Malt Extract Broth, and Sabouraud Dextrose Broth.

The term "inoculate" or "inoculation" as used herein refers to adding filamentous fungi into the bioreactor, thereby starting a filamentous fungi culture in the bioreactor.

The term "even distribution" of hydrogel beads refers to the even spreading of hydrogel beads on the at least one perforated plate of the bioreactor such that a layer of roughly uniform thickness is achieved, with roughly uniform spacing of the hydrogel beads within this layer. The hydrogel beads are evenly distributed in suspension, either by means of aeration or by means of stirring, and then let settle by gravity onto the perforated plate(s).

The term "aeration" as used herein refers to introducing sterilized air with a composition of 20-30 % O₂, 0.04-2 % CO₂, and 68-78.96% N₂ into the bioreactor. Aeration can be done through the use of a sparger (5).

The term "stirring" as used herein refers to mechanical bringing into motion of liquids or liquid-solid mixtures by means of a stirrer (see e.g. Figures 2 and 3), e.g. a stirrer blade, a stirring bar, or a stirrer shaft.

Aeration and stirring can also serve to tear produced filamentous fungi biomass (e.g. mycelium) that has been suspended in liquid by mechanical force. Tearing leads to the separation of larger clumps of filamentous fungi biomass into smaller pieces which are small enough in diameter to pass through the harvest port (4) or bead harvest port (8) of the bioreactor. The collection of torn filamentous fungi biomass, which can occur by flushing, i.e. by liquid flow, or by gravity through the harvest port is referred to as "harvesting" or "harvest".

"Drying" as used herein refers to reduction or removal of moisture from filamentous fungi biomass and/or hydrogel beads, e.g. by tray drying, fluid bed drying, spray drying, lyophilization.

In a first aspect, the invention provides a method of aseptically producing filamentous fungi in a bioreactor by pseudo-solid state fermentation, comprising the steps of:
(a) introduction of hydrogel beads into a chamber (1) of the bioreactor in solution via an inoculation port (2) or
   *in situ* production of hydrogel beads inside the chamber (1) of the bioreactor by introduction of at least a crosslinking agent and a biopolymer solution into the chamber (1) via an inoculation port (2), optionally wherein the biopolymer solution comprises nutrients, followed by washing of the produced beads;
(b) if no nutrients are comprised by the biopolymer solution in step (a), equilibration of the hydrogel beads with a growth medium comprising nutrients;
(c) inoculation of filamentous fungi into the chamber (1) via the inoculation port (2);
(d) even distribution of the inoculated hydrogel beads on at least one perforated plate (3) arranged horizontally within the chamber (1) by means of aeration or stirring;
(e) removal of all or virtually all liquids from the chamber (1) via a harvest port (4);
(f) fermentation of the filamentous fungi inside the bioreactor in the absence of any or virtually any liquids to increase filamentous fungi biomass, wherein the hydrogel beads are the only growth substrate and growth matrix inside the bioreactor;
(g) optionally at least one introduction of growth medium followed by repetition of steps (e)-(f) to re-equilibrate the hydrogel beads;
(h) introduction of liquid to resuspend the hydrogel beads and filamentous fungi biomass and tearing the filamentous fungi biomass by aeration or stirring;
(i) harvest of the filamentous fungi biomass and hydrogel beads via the harvest port (4) by flushing;
(j) optional drying of the harvested material.

In one embodiment, the hydrogel beads comprise at least one biopolymer and water. In one such embodiment, the at least one biopolymer is selected from the group consisting of: alginate, carrageenan, and gellan gum. In another such embodiment, the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%. In another such embodiment, the at least one biopolymer is selected from the group consisting of: alginate, carrageenan, and gellan gum and the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%. In a preferred such embodiment, the at least one biopolymer is alginate. In an especially preferred such embodiment, the at least one biopolymer is alginate and the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%.

In an embodiment, the hydrogel beads further comprise a filler compound. A "filler compound" is a mineral or organic material that increases the stability of hydrogel beads. Preferably, the filler compound content of the hydrogel beads is no more than 50%. In one such embodiment, the filler compound is selected from the group consisting of: talc, corn starch, corn flour, betonite, kaolinite, quartz silica powder, peat, arabic gum. That is, in an embodiment, the at least one biopolymer is selected from the group consisting of: alginate, carrageenan, and gellan gum and the filler compound is selected from the group consisting of: talc, corn starch, corn flour, betonite, kaolinite, quartz silica powder, peat, arabic gum. In another such embodiment, the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97% and the filler compound is selected from the group consisting of: talc, corn starch, corn flour, betonite, kaolinite, quartz silica powder, peat, arabic gum. In another such embodiment, the at least one biopolymer is selected from the group consisting of: alginate, carrageenan, and gellan gum, the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%, and the filler compound is selected from the group consisting of: talc, corn starch, corn flour, betonite, kaolinite, quartz silica powder, peat, arabic gum. In a preferred such embodiment, the at least one biopolymer is alginate and the filler compound is selected from the group consisting of: talc, corn starch, corn flour, betonite, kaolinite, quartz silica powder, peat, arabic gum. In an especially preferred such embodiment, the at least one biopolymer is alginate, the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%, and the filler compound is selected from the group consisting of: talc, corn starch, corn flour, betonite, kaolinite, quartz silica powder, peat, arabic gum.

In an embodiment, in any of the inventive methods of the first aspect, the hydrogel beads have a size of 0.1mm to 6mm. In a preferred embodiment, the hydrogel beads have a size of 0.5 to 5mm. In an especially preferred embodiment, the hydrogel beads have a size of 2mm to 4mm.

In an embodiment, in any of the above methods of the invention, the hydrogel beads form a layer with a height of 1cm to 150cm, preferably of 2cm to 50cm, most preferably of 5cm to 20cm.

In an embodiment, in any of the inventive methods of the first aspect, the hydrogel beads comprise at least one carbon source. In a preferred embodiment, the at least one carbon source is selected from the group consisting of: a sugar, and a starch. In one such embodiment, the sugar is selected from the group consisting of: monosaccharides and disaccharides. In another such embodiment, the starch is selected from the group consisting of: oligosaccharides and polysaccharides.

In an embodiment, in any of the inventive methods of the first aspect, inoculation with filamentous fungi is inoculation with spores.

In an embodiment, in any of the inventive methods of the first aspect, equilibration occurs for 1 to 28 hours. In a preferred embodiment, equilibration occurs for 18 to 28 hours. In an especially preferred embodiment, equilibration occurs for 24 hours.

In an embodiment, in any of the inventive methods of the first aspect, during fermentation, the humidity inside the chamber (1) is controlled by aeration via a sparger (5). In a preferred embodiment, the humidity inside the chamber (1) is maintained at between 25% and 100%. In an especially preferred embodiment, the humidity inside the chamber (1) is maintained at between 70% and 100%.

In an embodiment, in any of the inventive methods of the first aspect, fermentation occurs for 6 hours to 120 days. In a preferred aspect, fermentation occurs for 1 to 90 days.

In an embodiment, in any of the inventive methods of the first aspect, the filamentous fungi are selected from the list consisting of: Zygomycota, Ascomycota, Basidiomycota and Glomeromycota; preferably from the list consisting of: *Ampelomyces, Aspergillus, Beauveria, Fusarium, Isaria, Lecanicillium, Metarhizium, Penicillium, Phoma, Purpureocillium, Serendipita, Trichoderma, Verticillium and* Mycorrhiza. In another embodiment, the filamentous fungi are ectomycorrhiza or arbuscular mycorrhiza (AMF).

In a second aspect, the invention provides a method of aseptically producing filamentous fungi in a bioreactor by pseudo-solid state fermentation, comprising the steps of:
(a) introduction of hydrogel beads comprising viable filamentous fungi into a chamber (1) of the bioreactor in solution via an inoculation port (2) or
   *in situ* production of hydrogel beads inside the chamber (1) of the bioreactor by introduction of at least filamentous fungi, a crosslinking agent, and a biopolymer solution into the chamber (1) via an inoculation port (2), optionally wherein the biopolymer solution comprises nutrients, followed by washing of the produced beads;
(b) if no nutrients are comprised by the biopolymer solution in step (a), equilibration of the hydrogel beads with a growth medium comprising nutrients;
(c) even distribution of the hydrogel beads on at least one perforated plate (3) arranged horizontally within the chamber (1) by means of aeration or stirring;
(d) removal of all or virtually all liquids from the chamber (1) via a harvest port (4);
(e) fermentation of the filamentous fungi inside the bioreactor in the absence of any or virtually liquids to increase filamentous fungi biomass, wherein the hydrogel beads are the only growth substrate and growth matrix inside the bioreactor;
(f) optionally at least one introduction of growth medium followed by repetition of steps (d)-(e) to re-equilibrate the hydrogel beads;
(g) introduction of liquid to resuspend the hydrogel beads and filamentous fungi biomass and tearing the filamentous fungi biomass by aeration or stirring;
(h) harvest of the filamentous fungi biomass and hydrogel beads via the harvest port (4) by flushing;
(i) optional drying of the harvested material.

In one embodiment, the hydrogel beads comprise at least one biopolymer and water. In one such embodiment, the at least one biopolymer is selected from the group consisting of: alginate, carrageenan, and gellan gum. In another such embodiment, the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%. In another such embodiment, the at least one biopolymer is selected from the group consisting of: alginate, carrageenan, and gellan gum and the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%. In a preferred such embodiment, the at least one biopolymer is alginate. In an especially preferred such embodiment, the at least one biopolymer is alginate and the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%.

In an embodiment, the hydrogel beads further comprise a filler compound. In one such embodiment, the filler compound is selected from the group consisting of: talc, corn starch, corn flour, betonite, kaolinite, quartz silica powder, peat, arabic gum. That is, in an embodiment, the at least one biopolymer is selected from the group consisting of: alginate, carrageenan, and gellan gum and the filler compound is selected from the group consisting of: talc, corn starch, corn flour, betonite, kaolinite, quartz silica powder, peat, arabic gum. In another such embodiment, the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97% and the filler compound is selected from the group consisting of: talc, corn starch, corn flour, betonite, kaolinite, quartz silica powder, peat, arabic gum. In another such embodiment, the at least one biopolymer is selected from the group consisting of: alginate, carrageenan, and gellan gum, the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%, and the filler compound is selected from the group consisting of: talc, corn starch, corn flour, betonite, kaolinite, quartz silica powder, peat, arabic gum. In a preferred such embodiment, the at least one biopolymer is alginate and the filler compound is selected from the group consisting of: talc, corn starch, corn flour, betonite, kaolinite, quartz silica powder, peat, arabic gum. In an especially preferred such embodiment, the at least one biopolymer is alginate, the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%, and the filler compound is selected from the group consisting of: talc, corn starch, corn flour, betonite, kaolinite, quartz silica powder, peat, arabic gum.

In an embodiment, in any of the inventive methods of the second aspect, the hydrogel beads have a size of 0.1mm to 6mm. In a preferred embodiment, the hydrogel beads have a size of 0.5 to 5mm. In an especially preferred embodiment, the hydrogel beads have a size of 2mm to 4mm.

In an embodiment, in any of the above methods of the invention, the hydrogel beads form a layer with a height of 1cm to 150cm, preferably of 2cm to 25cm, most preferably of 5cm to 20cm.

In an embodiment, in any of the inventive methods of the second aspect, the hydrogel beads comprise at least one carbon source. In a preferred embodiment, the at least one carbon source is selected from the group consisting of: a sugar, and a starch. In one such embodiment, the sugar is selected from the group consisting of: monosaccharides and disaccharides. In another such embodiment, the starch is selected from the group consisting of: oligosaccharides and polysaccharides.

In an embodiment, in any of the inventive methods of the second aspect, inoculation with filamentous fungi is inoculation with spores.

In an embodiment, in any of the inventive methods of the second aspect, equilibration occurs for 1 to 28 hours. In a preferred embodiment, equilibration occurs for 18 to 28 hours. In an especially preferred embodiment, equilibration occurs for 24 hours.

In an embodiment, in any of the inventive methods of the second aspect, during fermentation, the humidity inside the chamber (1) is controlled by aeration via a sparger (5). In a preferred embodiment, the humidity inside the chamber (1) is maintained at between 25% and 100%. In an especially preferred embodiment, the humidity inside the chamber (1) is maintained at between 70% and 100%.

In an embodiment, in any of the inventive methods of the second aspect, fermentation occurs for 6 hours to 120 days. In a preferred aspect, fermentation occurs for 1 to 90 days.

In an embodiment, in any of the inventive methods of the second aspect, the filamentous fungi are selected from the list consisting of: Zygomycota, Ascomycota, Basidiomycota and Glomeromycota, preferably from the list consisting of: *Ampelomyces, Aspergillus, Beauveria, Fusarium, Isaria, Lecanicillium, Metarhizium, Penicillium, Phoma, Purpureocillium, Serendipita, Trichoderma, Verticillium and* Mycorrhiza. In another embodiment, the filamentous fungi are ectomycorrhiza or arbuscular mycorrhiza (AMF).

In a third aspect, the invention provides a method of continuously, aseptically producing filamentous fungi in a bioreactor by pseudo-solid state fermentation, comprising the steps of:
(a) Introduction of hydrogel beads into a chamber (1) of the bioreactor in solution via an inoculation port (2) or
   *in situ* production of hydrogel beads inside the chamber (1) of the bioreactor by introduction of at least a crosslinking agent and a biopolymer solution into the chamber (1) via an inoculation port (2), optionally wherein the biopolymer solution comprises nutrients, followed by washing of the produced beads;
(b) if no nutrients are comprised by the biopolymer solution in step (a), equilibration of the inoculated hydrogel beads with a growth medium comprising nutrients;
(c) inoculation of filamentous fungi into the chamber (1) via the inoculation port (2);
(d) even distribution of the inoculated hydrogel beads on at least one perforated plate (3) arranged horizontally within the chamber (1) by means of aeration or stirring;
(e) removal of all or virtually all liquids from the chamber (1) via a harvest port (4);
(f) fermentation of the filamentous fungi inside the bioreactor in the absence of any or virtually any liquids to increase filamentous fungi biomass, wherein the hydrogel beads are the only growth substrate and growth matrix inside the bioreactor;
(g) optionally at least one introduction of growth medium followed by repetition of steps (e)-(f) to re-equilibrate the hydrogel beads;
(h) introduction of liquid to resuspend the hydrogel beads and filamentous fungi biomass and tearing the filamentous fungi biomass by aeration or stirring;
(i) draining liquid and suspended filamentous fungi biomass via the harvest port (4), wherein the harvest port comprises or is covered by a further perforated plate, wherein the openings in the further perforated plate have a smaller diameter than the hydrogel beads such that the hydrogel beads are retained in the chamber (1);
(j) equilibration of the hydrogel beads with a growth medium;
(k) even distribution of the hydrogel beads and any remaining filamentous fungi biomass on the at least one perforated plate (3) arranged horizontally within the chamber (1) by means of aeration or stirring;
(l) continuous repetition of steps (e)-(k);
(m) optional drying of the harvested material.

In one embodiment, the hydrogel beads comprise at least one biopolymer and water. In one such embodiment, the at least one biopolymer is selected from the group consisting of: alginate, carrageenan, and gellan gum. In another such embodiment, the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%. In another such embodiment, the at least one biopolymer is selected from the group consisting of: alginate, carrageenan, and gellan gum and the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%. In a preferred such embodiment, the at least one biopolymer is alginate. In an especially preferred such embodiment, the at least one biopolymer is alginate and the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%.

In an embodiment, the hydrogel beads further comprise a filler compound. In one such embodiment, the filler compound is selected from the group consisting of: talc, corn starch, corn flour, betonite, kaolinite, quartz silica powder, peat, arabic gum. That is, in an embodiment, the at least one biopolymer is selected from the group consisting of: alginate, carrageenan, and gellan gum and the filler compound is selected from the group consisting of: talc, corn starch, corn flour, betonite, kaolinite, quartz silica powder, peat, arabic gum. In another such embodiment, the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97% and the filler compound is selected from the group consisting of: talc, corn starch, corn flour, betonite, kaolinite, quartz silica powder, peat, arabic gum. In another such embodiment, the at least one biopolymer is selected from the group consisting of: alginate, carrageenan, and gellan gum, the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%, and the filler compound is selected from the group consisting of: talc, corn starch, corn flour, betonite, kaolinite, quartz silica powder, peat, arabic gum. In a preferred such embodiment, the at least one biopolymer is alginate and the filler compound is selected from the group consisting of: talc, corn starch, corn flour, betonite, kaolinite, quartz silica powder, peat, arabic gum. In an especially preferred such embodiment, the at least one biopolymer is alginate, the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%, and the filler compound is selected from the group consisting of: talc, corn starch, corn flour, betonite, kaolinite, quartz silica powder, peat, arabic gum.

In an embodiment, in any of the inventive methods of the third aspect, the hydrogel beads have a size of 0.1mm to 6mm. In a preferred embodiment, the hydrogel beads have a size of 0.5 to 5mm. In an especially preferred embodiment, the hydrogel beads have a size of 2mm to 4mm.

In an embodiment, in any of the above methods of the invention, the hydrogel beads form a layer with a height of 1cm to 150cm, preferably of 2cm to 25cm, most preferably of 5cm to 20cm.

In an embodiment, in any of the inventive methods of the third aspect, the hydrogel beads comprise at least one carbon source. In a preferred embodiment, the at least one carbon source is selected from the group consisting of: a sugar, and a starch. In one such embodiment, the sugar is selected from the group consisting of: monosaccharides and disaccharides. In another such embodiment, the starch is selected from the group consisting of: oligosaccharides and polysaccharides.

In an embodiment, in any of the inventive methods of the third aspect, inoculation with filamentous fungi is inoculation with spores.

In an embodiment, in any of the inventive methods of the third aspect, equilibration occurs for 1 to 28 hours. In a preferred embodiment, equilibration occurs for 18 to 28 hours. In an especially preferred embodiment, equilibration occurs for 24 hours.

In an embodiment, in any of the inventive methods of the third aspect, during fermentation, the humidity inside the chamber (1) is controlled by aeration via a sparger (5). In a preferred embodiment, the humidity inside the chamber (1) is maintained at between 25% and 100%. In an especially preferred embodiment, the humidity inside the chamber (1) is maintained at between 70% and 100%.

In an embodiment, in any of the inventive methods of the third aspect, fermentation occurs for 6 hours to 120 days. In a preferred aspect, fermentation occurs for 1 to 90 days.

In an embodiment, in any of the inventive methods of the third aspect, the filamentous fungi are selected from the list consisting of: Zygomycota, Ascomycota, Basidiomycota and Glomeromycota, preferably from the list consisting of: *Ampelomyces, Aspergillus, Beauveria, Fusarium, Isaria, Lecanicillium, Metarhizium, Penicillium, Phoma, Purpureocillium, Serendipita, Trichoderma, Verticillium and* Mycorrhiza.

In a fourth aspect, the invention provides a method of continuously, aseptically producing filamentous fungi in a bioreactor by pseudo-solid state fermentation, comprising the steps of:
(a) Introduction of hydrogel beads comprising viable filamentous fungi into a chamber (1) of the bioreactor in solution via an inoculation port (2) or
   *in situ* production of hydrogel beads inside the chamber (1) of the bioreactor by introduction of at least filamentous fungi, a crosslinking agent, and a biopolymer solution into the chamber (1) via an inoculation port (2), optionally wherein the biopolymer solution comprises nutrients, followed by washing of the produced beads;
(b) if no nutrients are comprised by the biopolymer solution in step (a), equilibration of the hydrogel beads with a growth medium comprising nutrients;
(c) even distribution of the hydrogel beads on at least one perforated plate (3) arranged horizontally within the chamber (1) by means of aeration;
(d) removal of all or virtually all liquids from the chamber (1) via a harvest port (4);
(e) fermentation of the filamentous fungi inside the bioreactor in the absence of any or virtually any liquids to increase filamentous fungi biomass, wherein the hydrogel beads are the only growth substrate and growth matrix inside the bioreactor;
(f) optionally at least one introduction of growth medium followed by repetition of steps (d)-(e) to re-equilibrate the hydrogel beads;
(g) introduction of liquid to resuspend the hydrogel beads and filamentous fungi biomass and tearing the filamentous fungi biomass by aeration or stirring;
(h) draining liquid and suspended filamentous fungi biomass via the harvest port (4), wherein the harvest port comprises or is covered by a further perforated plate, wherein the openings in the further perforated plate have a smaller diameter than the hydrogel beads such that the hydrogel beads are retained in the chamber (1);
(i) equilibration of the hydrogel beads with a growth medium;
(j) even distribution of the inoculated hydrogel beads and any remaining filamentous fungi biomass on the at least one perforated plate (3) arranged horizontally within the chamber (1) by means of aeration or stirring;
(k) continuous repetition of steps (e)-(j);
(l) optional drying of the harvested material.

In one embodiment, the hydrogel beads comprise at least one biopolymer and water. In one such embodiment, the at least one biopolymer is selected from the group consisting of: alginate, carrageenan, and gellan gum. In another such embodiment, the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%. In another such embodiment, the at least one biopolymer is selected from the group consisting of: alginate, carrageenan, and gellan gum and the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%. In a preferred such embodiment, the at least one biopolymer is alginate. In an especially preferred such embodiment, the at least one biopolymer is alginate and the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%.

In an embodiment, the hydrogel beads further comprise a filler compound. In one such embodiment, the filler compound is selected from the group consisting of: talc, corn starch, corn flour, betonite, kaolinite, quartz silica powder, peat, arabic gum. That is, in an embodiment, the at least one biopolymer is selected from the group consisting of: alginate, carrageenan, and gellan gum and the filler compound is selected from the group consisting of: talc, corn starch, corn flour, betonite, kaolinite, quartz silica powder, peat, arabic gum. In another such embodiment, the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97% and the filler compound is selected from the group consisting of: talc, corn starch, corn flour, betonite, kaolinite, quartz silica powder, peat, arabic gum. In another such embodiment, the at least one biopolymer is selected from the group consisting of: alginate, carrageenan, and gellan gum, the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%, and the filler compound is selected from the group consisting of: talc, corn starch, corn flour, betonite, kaolinite, quartz silica powder, peat, arabic gum. In a preferred such embodiment, the at least one biopolymer is alginate and the filler compound is selected from the group consisting of: talc, corn starch, corn flour, betonite, kaolinite, quartz silica powder, peat, arabic gum. In an especially preferred such embodiment, the at least one biopolymer is alginate, the water content of the hydrogel beads is at least 50%, preferably above 80%, e.g. 80%, 81%, 82%, 83%, 84%, 85%, 86%, 87%, 88%, 90%, 91%, 92%, 93%, 94%, 95%, 96% or 97%, and the filler compound is selected from the group consisting of: talc, corn starch, corn flour, betonite, kaolinite, quartz silica powder, peat, arabic gum.

In an embodiment, in any of the inventive methods of the fourth aspect, the hydrogel beads have a size of 0.1mm to 6mm. In a preferred embodiment, the hydrogel beads have a size of 0.5 to 5mm. In an especially preferred embodiment, the hydrogel beads have a size of 2mm to 4mm.

In an embodiment, in any of the above methods of the invention, the hydrogel beads form a layer eith a height of 1cm to 150cm, preferably of 2cm to 25cm, most preferably of 5cm to 20cm.

In an embodiment, in any of the inventive methods of the fourth aspect, the hydrogel beads comprise at least one carbon source. In a preferred embodiment, the at least one carbon source is selected from the group consisting of: a sugar, and a starch. In one such embodiment, the sugar is selected from the group consisting of: monosaccharides and disaccharides. In another such embodiment, the starch is selected from the group consisting of: oligosaccharides and polysaccharides.

In an embodiment, in any of the inventive methods of the fourth aspect, inoculation with filamentous fungi is inoculation with spores.

In an embodiment, in any of the inventive methods of the fourth aspect, equilibration occurs for 1 to 28 hours. In a preferred embodiment, equilibration occurs for 18 to 28 hours. In an especially preferred embodiment, equilibration occurs for 24 hours.

In an embodiment, in any of the inventive methods of the fourth aspect, during fermentation, the humidity inside the chamber (1) is controlled by aeration via a sparger (5). In a preferred embodiment, the humidity inside the chamber (1) is maintained at between 25% and 100%. In an especially preferred embodiment, the humidity inside the chamber (1) is maintained at between 70% and 100%.

In an embodiment, in any of the inventive methods of the fourth aspect, fermentation occurs for 6 hours to 120 days. In a preferred aspect, fermentation occurs for 1 to 90 days.

In an embodiment, in any of the inventive methods of the fourth aspect, the filamentous fungi are selected from the list consisting of: Zygomycota, Ascomycota, Basidiomycota and Glomeromycota, preferably from the list consisting of: *Ampelomyces, Aspergillus, Beauveria, Fusarium, Isaria, Lecanicillium, Metarhizium, Penicillium, Phoma, Purpureocillium, Serendipita, Trichoderma, Verticillium and* Mycorrhiza.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

The detailed description is merely exemplary in nature and is not intended to limit application and uses. The following examples further illustrate the present invention without, however, limiting the scope of the invention thereto. Various changes and modifications can be made by those skilled in the art on the basis of the description of the invention, and such changes and modifications are also included in the present invention.

### EXAMPLES

### 1. General protocol for aseptic mass production of conidial spores of a filamentous fungi from the family of Cordycipitaceae in PSSF in lab scale.

A lab scale glass bioreactor (DasGip) with a recommended working volume of 1 litre is used for the cultivation of filamentous fungi. The inner design of the bioreactor resemblesFigure 2 . The bioreactor comprises a chamber with a diameter of about 13 cm and a maximal height of about 20 cm (1), an inoculation port with a diameter of about 3 cm (2), a perforated polypropylene plate with round holes of about 3mm diameter and an open area of 33% (3), a harvest port for liquid in the form of a silicone tube that reaches from the top to the bottom of the vessel (4), a ring sparger with a diameter of about 8cm (5), and a bead harvest port with a diameter of about 3 cm (8). Furthermore, the bioreactor contains a condenser for the exhaust gas.

The bioreactor is filled with about 600ml of potato dextrose broth (PDB) and is sterilized inside an autoclave as a whole for 30min at 121°C. During autoclaving, the lid of the vessel is loosely closed to prevent overpressure and is closed immediately after autoclaving.

250g of hydrogel beads with a diameter of about 4mm are formed by dripping an autoclaved 2% alginate solution containing PDB into a 2% autoclaved CaCl₂ solution. After at least one hour within the CaCl₂ solution, the beads are rinsed with sterile water within a sterile workbench and transferred to the bioreactor via the inoculation port (2).

Within the bioreactor the beads settle down on the perforated plate (4) where they are covered with liquid medium.

The inoculation of the bioreactor is performed inside the sterile workbench with 5ml of spore suspension at a concentration of 2x10⁷ spores/ml via the inoculation port (2) into the liquid on top of the bead layer.

After inoculation, the bioreactor is connected to the control tower where an aeration of 1,5L/h is adjusted.

After 24 hours of incubation in liquid, all the liquid media is removed via the harvest port (4). Consecutive cultivation without liquid is performed for about 7 days, where after the layer of hydrogel beads is covered with a dense mixture of hyphae and spores.

For a liquid harvest of spores, the bioreactor is filled with 600ml of a 1% Tween 20 solution, the stirrer is turned on to about 100rpm and aeration is increased to 30L/h to tear the mycelium. The homogeneous bioreactor content can be harvested via the bead harvest port (8). Approximately 10¹¹ aseptically produced and harvested conidia spores can be expected following this procedure. The Spores can be separated from beads and mycelium via filtration.

Alternatively, the mixture of spores and beads can be dried to a final concentration of about 5x10⁹ spores per g product with a residual humidity of 10%.

### 2. Reference production of conidial spores of a filamentous fungi from the family of Cordycipitaceae in SSF on barley in lab scale

A lab scale glass bioreactor (DasGip) with a recommended working volume of 1 litre is used for the cultivation of filamentous fungi. The inner design of the bioreactor resembles FFigure 2 with the exception of not having a stirrer. The bioreactor comprises a chamber with a diameter of about 13 cm and a maximal height of about 20 cm (1), an inoculation port with a diameter of about 3 cm (2), a perforated polypropylene plate with round holes of about 3mm diameter and an open area of 33% (3), a harvest port for liquid in the form of a silicone tube that reaches from the top to the bottom of the vessel (4), a ring sparger with a diameter of about 8cm (5), and a bead harvest port with a diameter of about 3 cm (8). Furthermore, the bioreactor contains a condenser for the exhaust gas.

The Bioreactor is filled with 100 ml of PD media and 140 g barley (30% wet basis moisture content) and is sterilized inside an autoclave as a whole for 30min at 121°C. During autoclaving, the lid of the vessel is loosely closed to prevent overpressure and is closed immediately after autoclaving. A 1 litre Schott bottle containing 500 ml of PD broth is sterilized following the same autoclaving procedure. The 500 ml of autoclaved PD broth are added to the autoclaved bioreactor via the inoculation port (1) to a total of 600ml PD broth.

The inoculation of the bioreactor is performed inside the sterile workbench with 5ml of spore suspension at a concentration of 2x10⁷ spores/ml via the inoculation port (2) into the liquid on top of the bead layer.

After inoculation, the bioreactor is connected to the control tower where an aeration of 1,5L/h is adjusted.

After 24 hours of incubation in liquid, all the liquid media is removed via the harvest port (4). The remaining barley layer covers a volume of about 300ml. Consecutive cultivation without liquid is performed for about 7 days, where after the whole layer of barley is infused with a mixture of mycelium and spores resulting in a dense and robust layer of barley.

For spore harvesting the robust barley layer is removed from the bioreactor via opening the top lid of the bioreactor. Thereafter, the barley layer is cut into of about 10cm³ pieces using a sharp knife. 600ml of a 1% Tween 20 solution, is added to the cut barley pieces. Approximately 10¹¹ conidia spores can be expected following this procedure.

The spores can be separated from the barley pieces and mycelium via filtration. Alternatively, the mixture of spores, mycelium and barley pieces can be dried to a final concentration of about 2x10⁹ spores per g product with a residual humidity of about 10%.

Although approximately the same number of spores 10¹¹ can be harvested from PSSF (example 1) and SSF (example 2), the concentration of the final dried product is two to ten times higher in case of PSSF compared to SSF.

### 3. Reference production of conidial spores of a filamentous fungi from the family of Cordycipitaceae in submerse fermentation (SmF) in lab scale utilizing encapsulated spores as inoculum

A lab scale glass bioreactor (DasGip) with a recommended working volume of 1 litre is used for the cultivation of filamentous fungi. The inner design of the bioreactor resemblesFigure 2 with the exception of not containing a perforated plate (3). The bioreactor comprises a chamber with a diameter of about 13 cm and a maximal height of about 20 cm (1), an inoculation port with a diameter of about 3 cm (2), a harvest port for liquid in the form of a silicone tube that reaches from the top to the bottom of the vessel (4), a ring sparger with a diameter of about 8cm (5), and a bead harvest port with a diameter of about 3 cm (8). Furthermore, the bioreactor contains a condenser for the exhaust gas.

The bioreactor is filled with about 500ml of liquid potato dextrose broth (PDB) and is sterilized inside an autoclave as a whole for 30min at 121°C. During autoclaving, the lid of the vessel is loosely closed to prevent overpressure and is closed immediately after autoclaving.

For inoculation of the bioreactor spores are encapsulated into hydrogel beads, therefore 50g of hydrogel beads with a diameter of about 4mm are formed by dripping an autoclaved 2% alginate solution containing 5ml of spore suspension at a concentration of 2x10⁷ Spores/ml and PDB into a 2% autoclaved CaCl₂ solution. After at least one hour within the CaCl₂ solution, the beads are rinsed with sterile water within a sterile workbench and transferred to the bioreactor via the inoculation port (2).

After inoculation, the bioreactor is connected to the control tower where an aeration of 3,6L/h is adjusted and the stirrer is set to 100rpm.

After 8 days of incubation in liquid, the hydrogel beads are covered by a layer of mycelium and spores. Beads and liquid media are removed via the harvest port (4).

600ml of a 1% Tween 20 solution, is added to the hydrogel bead, mycelia and spore suspension.

Approximately 2x10⁹ conidia spores can be expected following this procedure, which is significantly lower than the setups using PSSF (example 1) and SSF (example 2) with approximately 10¹¹ final spores each.

## Claims

1. A method of aseptically producing filamentous fungi in a bioreactor by pseudo-solid state fermentation, comprising the steps of:
(a) introduction of hydrogel beads into a chamber (1) of the bioreactor in solution via an inoculation port (2) or
*in situ* production of hydrogel beads inside the chamber (1) of the bioreactor by introduction of at least a crosslinking agent and a biopolymer solution into the chamber (1) via an inoculation port (2), optionally wherein the biopolymer solution comprises nutrients, followed by washing of the produced beads;
(b) if no nutrients are comprised by the biopolymer solution in step (a), equilibration of the hydrogel beads with a growth medium comprising nutrients;
(c) inoculation of filamentous fungi into the chamber (1) via the inoculation port (2);
(d) even distribution of the inoculated hydrogel beads on at least one perforated plate (3) arranged horizontally within the chamber (1) by means of aeration or stirring;
(e) removal of all or virtually all liquids from the chamber (1) via a harvest port (4);
(f) fermentation of the filamentous fungi inside the bioreactor in the absence of any or virtually any liquids to increase filamentous fungi biomass, wherein the hydrogel beads are the only growth substrate and growth matrix inside the bioreactor;
(g) optionally at least one introduction of growth medium followed by repetition of steps (e)-(f) to re-equilibrate the hydrogel beads;
(h) introduction of liquid to resuspend the hydrogel beads and filamentous fungi biomass and tearing the filamentous fungi biomass by aeration or stirring;
(i) harvest of the filamentous fungi biomass and hydrogel beads via the harvest port (4) by flushing;
(j) optional drying of the harvested material.

2. A method of aseptically producing filamentous fungi in a bioreactor by pseudo-solid state fermentation, comprising the steps of:
(a) introduction of hydrogel beads comprising viable filamentous fungi into a chamber (1) of the bioreactor in solution via an inoculation port (2) or
*in situ* production of hydrogel beads inside the chamber (1) of the bioreactor by introduction of at least filamentous fungi, a crosslinking agent, and a biopolymer solution into the chamber (1) via an inoculation port (2), optionally wherein the biopolymer solution comprises nutrients, followed by washing of the produced beads;
(b) if no nutrients are comprised by the biopolymer solution in step (a), equilibration of the hydrogel beads with a growth medium comprising nutrients;
(c) even distribution of the hydrogel beads on at least one perforated plate (3) arranged horizontally within the chamber (1) by means of aeration or stirring;
(d) removal of all or virtually all liquids from the chamber (1) via a harvest port (4);
(e) fermentation of the filamentous fungi inside the bioreactor in the absence of any or virtually any liquids to increase filamentous fungi biomass, wherein the hydrogel beads are the only growth substrate and growth matrix inside the bioreactor;
(f) optionally at least one introduction of growth medium followed by repetition of steps (d)-(e) to re-equilibrate the hydrogel beads;
(g) introduction of liquid to resuspend the hydrogel beads and filamentous fungi biomass and tearing the filamentous fungi biomass by aeration or stirring;
(h) harvest of the filamentous fungi biomass and hydrogel beads via the harvest port (4) by flushing;
(i) optional drying of the harvested material.

3. A method of continuously, aseptically producing filamentous fungi in a bioreactor by pseudo-solid state fermentation, comprising the steps of:
(a) Introduction of hydrogel beads into a chamber (1) of the bioreactor in solution via an inoculation port (2) or
*in situ* production of hydrogel beads inside the chamber (1) of the bioreactor by introduction of at least a crosslinking agent and a biopolymer solution into the chamber (1) via an inoculation port (2), optionally wherein the biopolymer solution comprises nutrients, followed by washing of the produced beads;
(b) if no nutrients are comprised by the biopolymer solution in step (a), equilibration of the inoculated hydrogel beads with a growth medium comprising nutrients;
(c) inoculation of filamentous fungi into the chamber (1) via the inoculation port (2);
(d) even distribution of the inoculated hydrogel beads on at least one perforated plate (3) arranged horizontally within the chamber (1) by means of aeration or stirring;
(e) removal of all or virtually all liquids from the chamber (1) via a harvest port (4);
(f) fermentation of the filamentous fungi inside the bioreactor in the absence of any or virtually any liquids to increase filamentous fungi biomass, wherein the hydrogel beads are the only growth substrate and growth matrix inside the bioreactor;
(g) optionally at least one introduction of growth medium followed by repetition of steps (e)-(f) to re-equilibrate the hydrogel beads;
(h) introduction of liquid to resuspend the hydrogel beads and filamentous fungi biomass and tearing the filamentous fungi biomass by aeration or stirring;
(i) draining liquid and suspended filamentous fungi biomass via the harvest port (4), wherein the harvest port comprises or is covered by a further perforated plate, wherein the openings in the further perforated plate have a smaller diameter than the hydrogel beads such that the hydrogel beads are retained in the chamber (1);
(j) equilibration of the hydrogel beads with a growth medium;
(k) even distribution of the hydrogel beads and any remaining filamentous fungi biomass on the at least one perforated plate (3) arranged horizontally within the chamber (1) by means of aeration or stirring;
(l) continuous repetition of steps (e)-(k);
(m) optional drying of the harvested material.

4. A method of continuously, aseptically producing filamentous fungi in a bioreactor by pseudo-solid state fermentation, comprising the steps of:
(a) Introduction of hydrogel beads comprising viable filamentous fungi into a chamber (1) of the bioreactor in solution via an inoculation port (2) or
*in situ* production of hydrogel beads inside the chamber (1) of the bioreactor by introduction of at least filamentous fungi, a crosslinking agent, and a biopolymer solution into the chamber (1) via an inoculation port (2), optionally wherein the biopolymer solution comprises nutrients, followed by washing of the produced beads;
(b) if no nutrients are comprised by the biopolymer solution in step (a), equilibration of the hydrogel beads with a growth medium comprising nutrients;
(c) even distribution of the hydrogel beads on at least one perforated plate (3) arranged horizontally within the chamber (1) by means of aeration;
(d) removal of all or virtually all liquids from the chamber (1) via a harvest port (4);
(e) fermentation of the filamentous fungi inside the bioreactor in the absence of any or virtually any liquids to increase filamentous fungi biomass, wherein the hydrogel beads are the only growth substrate and growth matrix inside the bioreactor;
(f) optionally at least one introduction of growth medium followed by repetition of steps (d)-(e) to re-equilibrate the hydrogel beads;
(g) introduction of liquid to resuspend the hydrogel beads and filamentous fungi biomass and tearing the filamentous fungi biomass by aeration or stirring;
(h) draining liquid and suspended filamentous fungi biomass via the harvest port (4), wherein the harvest port comprises or is covered by a further perforated plate, wherein the openings in the further perforated plate have a smaller diameter than the hydrogel beads such that the hydrogel beads are retained in the chamber (1);
(i) equilibration of the hydrogel beads with a growth medium;
(j) even distribution of the inoculated hydrogel beads and any remaining filamentous fungi biomass on the at least one perforated plate (3) arranged horizontally within the chamber (1) by means of aeration or stirring;
(k) continuous repetition of steps (e)-(j);
(l) optional drying of the harvested material.

5. The method of any one of claims 1 to 4, wherein the hydrogel beads comprise at least one biopolymer and water; preferably wherein the at least one biopolymer is selected from the group consisting of: alginate, carrageenan, and gellan gum and/or preferably wherein the water content of the hydrogel beads is at least 50%, preferaly at least 80%, more preferably at least 95%.

6. The method of claim 5, wherein the hydrogel beads further comprise a filler compound, preferably wherein the filler compound is selected from the group consisting of: talc, corn starch, corn flour, betonite, kaolinite, quartz silica powder, peat, arabic gum.

7. The method of any one of claims 1 to 6, wherein the hydrogel beads have a size of 0.1mm to 6mm, preferably of 0.5mm to 5mm, most preferably of 2mm to 4mm.

8. The method of any one of claims 1 to 7, wherein the hydrogel beads form a layer with a height of 1cm to 150cm, preferably of 2cm to 50cm, most preferably of 5cm to 20cm.

9. The method of any one of claims 1 to 8, wherein the hydrogel beads comprise at least one carbon source, preferably wherein the at least one carbon source is selected from the group consisting of: a sugar, and a starch; preferably wherein the sugar is selected from the group consisting of: monosaccharides and disaccharides, and wherein the starch is selected from the group consisting of: oligosaccharides and polysaccharides.

10. The method of any one of claims 1, 3, and 5 to 9, wherein inoculation with filamentous fungi is inoculation with spores.

11. The method of any one of claims 1 to 10, wherein equilibration occurs for 1 to 28 hours, preferably 18 to 28 hours, most preferably 24 hours.

12. The method of any one of claims 1 to 11, wherein during fermentation, the humidity inside the chamber (1) is controlled by aeration via a sparger (5), preferably wherein the humidity inside the chamber (1) is maintained at between 25% and 100%, preferably at between 70% and 100%.

13. The method of any one of claims 1 to 12, wherein fermentation occurs for 6 hours to 120 days, preferably for 1 to 90 days.

14. The method of any one of claims 1 to 13, wherein the filamentous fungi are selected from the group consisting of Zygomycota, Ascomycota, Basidiomycota and Glomeromycota, preferably from the list consisting of: *Ampelomyces, Aspergillus, Beauveria, Fusarium, Isaria, Lecanicillium, Metarhizium, Penicillium, Phoma, Purpureocillium, Serendipita, Trichoderma, Verticillium and* Mycorrhiza.

15. The method of claim 1 or 2, wherein the filamentous fungi are ectomycorrhiza or arbuscular mycorrhiza (AMF).

## Patentansprüche

1. Verfahren zur aseptischen Herstellung von Fadenpilzen in einem Bioreaktor durch Pseudo-Festphasenfermentation, umfassend die folgenden Schritte:
(a) Zugabe von Hydrogelkügelchen in eine Kammer (1) des Bioreaktors in Lösung über eine Inokulationsöffnung (2) oder
*in* situ-Herstellung von Hydrogelkügelchen innerhalb der Kammer (1) des Bioreaktors durch Zugabe mindestens eines Vernetzungsmittels und einer Biopolymerlösung in die Kammer (1) über eine Inokulationsöffnung (2), wobei die Biopolymerlösung optional Nährstoffe enthält, gefolgt von einem Waschen der hergestellten Kügelchen;
(b) falls die Biopolymerlösung in Schritt (a) keine Nährstoffe umfasst, Equilibrierung der Hydrogelkügelchen mit einem Nährstoffe enthaltenden Wachstumsmedium;
(c) Inokulation von Fadenpilzen in die Kammer (1) über die Inokulationsöffnung (2);
(d) gleichmäßige Verteilung der inokulierten Hydrogelkügelchen auf mindestens einer perforierten Platte (3), die horizontal innerhalb der Kammer (1) angeordnet ist, mittels Belüftung oder Rühren;
(e) Entfernen aller oder nahezu aller Flüssigkeiten aus der Kammer (1) über eine Entnahmeöffnung (4);
(f) Fermentierung der Fadenpilze im Inneren des Bioreaktors in Abwesenheit aller oder nahezu aller Flüssigkeiten, um die Biomasse der Fadenpilze zu erhöhen, wobei die Hydrogelkügelchen das einzige Wachstums-Substrat und die einzige Wachstumsmatrix im Inneren des Bioreaktors sind;
(g) optional mindestens eine Zugabe von Wachstumsmedium, gefolgt von einer Wiederholung der Schritte (e)-(f), um die Hydrogelkügelchen erneut zu equilibrieren;
(h) Zugabe von Flüssigkeit, um die Hydrogelkügelchen und die Biomasse der Fadenpilze wieder in Suspension zu bringen und Aufbrechen der Biomasse der Fadenpilze durch Belüftung oder Rühren;
(i) Ernte der Fadenpilzbiomasse und der Hydrogelkügelchen über die Entnahmestelle (4) durch Spülen;
(j) optionales Trocknen des geernteten Materials.

2. Verfahren zur aseptischen Herstellung von Fadenpilzen in einem Bioreaktor durch Pseudo-Festphasenfermentation, umfassend die folgenden Schritte:
(a) Zugabe von Hydrogelkügelchen, die lebensfähige Fadenpilze enthalten, in eine Kammer (1) des Bioreaktors in Lösung über eine Inokulationsöffnung (2) oder
*in* situ-Herstellung von Hydrogelkügelchen innerhalb der Kammer (1) des Bioreaktors durch Zugabe von mindestens Fadenpilzen, einem Vernetzungsmittel und einer Biopolymerlösung in die Kammer (1) über eine Inokulationsöffnung (2), wobei die Biopolymerlösung optional Nährstoffe enthält, gefolgt von einem Waschen der hergestellten Kügelchen;
(b) falls die Biopolymerlösung in Schritt (a) keine Nährstoffe enthält, Equilibrierung der Hydrogelkügelchen mit einem Nährstoffe enthaltenden Wachstumsmedium;
(c) gleichmäßige Verteilung der Hydrogelkügelchen auf mindestens einer perforierten Platte (3), die horizontal innerhalb der Kammer (1) angeordnet ist, mittels Belüftung oder Rühren;
(d) Entfernen aller oder nahezu aller Flüssigkeiten aus der Kammer (1) über eine Entnahmeöffnung (4);
(e) Fermentierung der Fadenpilze im Inneren des Bioreaktors in Abwesenheit aller oder nahezu aller Flüssigkeiten, um die Biomasse der Fadenpilze zu erhöhen, wobei die Hydrogelkügelchen das einzige Wachstums-Substrat und die einzige Wachstumsmatrix im Inneren des Bioreaktors sind;
(f) optional mindestens eine Zugabe von Wachstumsmedium, gefolgt von einer Wiederholung der Schritte (d)-(e), um die Hydrogelkügelchen erneut zu equilibrieren;
(g) Zugabe von Flüssigkeit, um die Hydrogelkügelchen und die Biomasse der Fadenpilze wieder in Suspension zu bringen und Aufbrechen der Biomasse der Fadenpilze durch Belüftung oder Rühren;
(h) Ernte der Fadenpilzbiomasse und der Hydrogelkügelchen über die Entnahmestelle (4) durch Spülen;
(i) optionales Trocknen des geernteten Materials.

3. Verfahren zur kontinuierlichen, aseptischen Herstellung von Fadenpilzen in einem Bioreaktor durch Pseudo-Festphasenfermentation, umfassend die folgenden Schritte:
(a) Zugabe von Hydrogelkügelchen in eine Kammer (1) des Bioreaktors in Lösung über eine Inokulationsöffnung (2) oder
*in* situ-Herstellung von Hydrogelkügelchen innerhalb der Kammer (1) des Bioreaktors durch Zugabe mindestens eines Vernetzungsmittels und einer Biopolymerlösung in die Kammer (1) über eine Inokulationsöffnung (2), wobei die Biopolymerlösung optional Nährstoffe enthält, gefolgt von einem Waschen der hergestellten Kügelchen;
(b) falls die Biopolymerlösung in Schritt (a) keine Nährstoffe enthält, Equilibrierung der inokulierten Hydrogelkügelchen mit einem Nährstoffe enthaltenden Wachstumsmedium;
(c) Inokulation von Fadenpilzen in die Kammer (1) über die Inokulationsöffnung (2);
(d) gleichmäßige Verteilung der inokulierten Hydrogelkügelchen auf mindestens einer perforierten Platte (3), die horizontal innerhalb der Kammer (1) angeordnet ist, mittels Belüftung oder Rühren;
(e) Entfernen aller oder nahezu aller Flüssigkeiten aus der Kammer (1) über eine Entnahmeöffnung (4);
(f) Fermentierung der Fadenpilze im Inneren des Bioreaktors in Abwesenheit aller oder nahezu aller Flüssigkeiten, um die Biomasse der Fadenpilze zu erhöhen, wobei die Hydrogelkügelchen das einzige Wachstums-Substrat und die einzige Wachstumsmatrix im Inneren des Bioreaktors sind;
(g) optional mindestens eine Zugabe von Wachstumsmedium, gefolgt von einer Wiederholung der Schritte (e)-(f), um die Hydrogelkügelchen erneut zu equilibrieren;
(h) Zugabe von Flüssigkeit, um die Hydrogelkügelchen und die Biomasse der Fadenpilze wieder in Suspension zu bringen, und Aufbrechen der Biomasse der Fadenpilze durch Belüftung oder Rühren;
(i) Ablassen der Flüssigkeit und der suspendierten Fadenpilzbiomasse über die Entnahmestelle (4), wobei die Entnahmestelle eine weitere perforierte Platte umfasst oder von dieser abgedeckt ist, wobei die Öffnungen in der weiteren perforierten Platte einen kleineren Durchmesser als die Hydrogelkügelchen aufweisen, so dass die Hydrogelkügelchen in der Kammer (1) zurückgehalten werden;
(j) Equilibrierung der Hydrogelkügelchen mit einem Wachstumsmedium;
(k) gleichmäßige Verteilung der Hydrogelkügelchen und der verbleibenden Fadenpilzbiomasse auf der mindestens einen perforierten Platte (3), die horizontal innerhalb der Kammer (1) angeordnet ist, mittels Belüftung oder Rühren;
(l) kontinuierliche Wiederholung der Schritte (e)-(k);
(m) optionales Trocknen des geernteten Materials.

4. Verfahren zur kontinuierlichen, aseptischen Herstellung von Fadenpilzen in einem Bioreaktor durch Pseudo-Festphasenfermentation, umfassend die folgenden Schritte:
(a) Zugabe von Hydrogelkügelchen, die lebensfähige Fadenpilze enthalten, in eine Kammer (1) des Bioreaktors in Lösung über eine Inokulationsöffnung (2) oder
*in* situ-Herstellung von Hydrogelkügelchen innerhalb der Kammer (1) des Bioreaktors durch Zugabe von mindestens Fadenpilzen, einem Vernetzungsmittel und einer Biopolymerlösung in die Kammer (1) über eine Inokulationsöffnung (2), wobei die Biopolymerlösung optional Nährstoffe enthält, gefolgt von einem Waschen der hergestellten Kügelchen;
(b) falls die Biopolymerlösung in Schritt (a) keine Nährstoffe enthält, Equilibrierung der Hydrogelkügelchen mit einem Nährstoffe enthaltenden Wachstumsmedium;
(c) gleichmäßige Verteilung der Hydrogelkügelchen auf mindestens einer perforierten Platte (3), die horizontal innerhalb der Kammer (1) angeordnet ist, mittels Belüftung;
(d) Entfernen aller oder nahezu aller Flüssigkeiten aus der Kammer (1) über eine Entnahmeöffnung (4);
(e) Fermentation der Fadenpilze im Bioreaktor in Abwesenheit aller oder nahezu aller Flüssigkeiten zur Steigerung der Biomasse der Fadenpilze, wobei die Hydrogelkügelchen das einzige Wachstumssubstrat und die einzige Wachstumsmatrix im Bioreaktor darstellen;
(f) optional mindestens eine Zugabe von Wachstumsmedium, gefolgt von einer Wiederholung der Schritte (d)-(e), um die Hydrogelkügelchen wieder ins Gleichgewicht zu bringen;
(g) Zugabe von Flüssigkeit, um die Hydrogelkügelchen und die Biomasse der Fadenpilze wieder in Suspension zu bringen, und Aufbrechen der Biomasse der Fadenpilze durch Belüftung oder Rühren;
(h) Ablassen der Flüssigkeit und der suspendierten Fadenpilzbiomasse über die Entnahmestelle (4), wobei die Entnahmestelle eine weitere perforierte Platte umfasst oder von dieser abgedeckt ist, wobei die Öffnungen in der weiteren perforierten Platte einen kleineren Durchmesser als die Hydrogelkügelchen aufweisen, so dass die Hydrogelkügelchen in der Kammer (1) zurückgehalten werden;
(i) Equilibrierung der Hydrogelkügelchen mit einem Wachstumsmedium;
(j) gleichmäßige Verteilung der inokulierten Hydrogelkügelchen und der verbleibenden Fadenpilzbiomasse auf der mindestens einen perforierten Platte (3), die horizontal innerhalb der Kammer (1) angeordnet ist, mittels Belüftung oder Rühren;
(k) kontinuierliche Wiederholung der Schritte (e)-(j);
(l) optionales Trocknen des geernteten Materials.

5. Verfahren nach einem der Ansprüche 1 bis 4, wobei die Hydrogelkügelchen mindestens ein Biopolymer und Wasser umfassen; vorzugsweise wobei das mindestens eine Biopolymer aus der Gruppe ausgewählt ist, bestehend aus: Alginat, Carrageen und Gellangummi, und/oder vorzugsweise wobei der Wassergehalt der Hydrogelkügelchen mindestens 50 %, vorzugsweise mindestens 80 %, noch bevorzugter mindestens 95 % beträgt.

6. Verfahren nach Anspruch 5, wobei die Hydrogelkügelchen ferner eine Füllstoffverbindung umfassen, vorzugsweise wobei die Füllstoffverbindung aus der Gruppe ausgewählt ist, bestehend aus: Talk, Maisstärke, Maismehl, Bentonit, Kaolinit, Quarzsilikatpulver, Torf, Gummi arabicum.

7. Verfahren nach einem der Ansprüche 1 bis 6, wobei die Hydrogelkügelchen eine Größe von 0.1 mm bis 6 mm, vorzugsweise von 0.5 mm bis 5 mm, am bevorzugtesten von 2 mm bis 4 mm aufweisen.

8. Verfahren nach einem der Ansprüche 1 bis 7, wobei die Hydrogelkügelchen eine Schicht mit einer Höhe von 1 cm bis 150 cm, vorzugsweise von 2 cm bis 50 cm, am bevorzugtesten von 5 cm bis 20 cm bilden.

9. Verfahren nach einem der Ansprüche 1 bis 8, wobei die Hydrogelkügelchen mindestens eine Kohlenstoffquelle umfassen, vorzugsweise wobei die mindestens eine Kohlenstoffquelle aus der Gruppe ausgewählt ist, bestehend aus: einem Zucker und einer Stärke; vorzugsweise wobei der Zucker aus der Gruppe ausgewählt ist, bestehend aus: Monosacchariden und Disacchariden, und wobei die Stärke aus der Gruppe ausgewählt ist, bestehend aus: Oligosacchariden und Polysacchariden.

10. Verfahren nach einem der Ansprüche 1, 3 und 5 bis 9, wobei die Inokulation mit Fadenpilzen eine Inokulation mit Sporen ist.

11. Verfahren nach einem der Ansprüche 1 bis 10, wobei die Equilibrierung 1 bis 28 Stunden, vorzugsweise 18 bis 28 Stunden, am bevorzugtesten 24 Stunden dauert.

12. Verfahren nach einem der Ansprüche 1 bis 11, wobei während der Fermentation die Luftfeuchtigkeit im Inneren der Kammer (1) durch Belüftung über einen Sprühkopf (5) kontrolliert wird, vorzugsweise wobei die Luftfeuchtigkeit im Inneren der Kammer (1) zwischen 25 % und 100 %, vorzugsweise zwischen 70 % und 100 %, gehalten wird.

13. Verfahren nach einem der Ansprüche 1 bis 12, wobei die Fermentation 6 Stunden bis 120 Tage, vorzugsweise 1 bis 90 Tage, dauert.

14. Verfahren nach einem der Ansprüche 1 bis 13, wobei die Fadenpilze aus der Gruppe ausgewählt sind, die aus Zygomycota, Ascomycota, Basidiomycota und Glomeromycota besteht, vorzugsweise aus der Liste, bestehend aus: *Ampelomyces, Aspergillus, Beauveria, Fusarium, Isaria, Lecanicillium, Metarhizium, Penicillium, Phoma, Purpureocillium, Serendipita, Trichoderma, Verticillium* und *Mycorrhiza.*

15. Verfahren nach Anspruch 1 oder 2, wobei die Fadenpilze Ektomykorrhiza oder arbuskuläre Mykorrhiza (AMF) sind.

## Revendications

1. Procédé de production aseptique de champignons filamenteux dans un bioréacteur par fermentation à l'état pseudo-solide, comprenant les étapes de :
(a) introduction de billes d'hydrogel dans une chambre (1) du bioréacteur en solution via un orifice d'inoculation (2) ou
production in situ de billes d'hydrogel à l'intérieur de la chambre (1) du bioréacteur par introduction d'au moins un agent de réticulation et une solution de biopolymère dans la chambre (1) via un orifice d'inoculation (2), facultativement sachant que la solution de biopolymère comprend des nutriments, suivie par un lavage des billes produites ;
(b) si aucun nutriment n'est compris dans la solution de biopolymère à l'étape (a), équilibrage des billes d'hydrogel avec un milieu de croissance comprenant des nutriments ;
(c) inoculation de champignons filamenteux dans la chambre (1) via l'orifice d'inoculation (2) ;
(d) distribution homogène des billes d'hydrogel inoculées sur au moins une plaque perforée (3) agencée horizontalement à l'intérieur de la chambre (1) par aération ou agitation ;
(e) élimination de tout ou pratiquement tout liquide de la chambre (1) via un orifice de récolte (4) ;
(f) fermentation des champignons filamenteux à l'intérieur du bioréacteur en l'absence de tout ou pratiquement tout liquide pour augmenter la biomasse de champignons filamenteux, sachant que les billes d'hydrogel sont le seul substrat de croissance et la seule matrice de croissance à l'intérieur du bioréacteur ;
(g) facultativement, au moins une introduction de milieu de croissance suivie par une répétition des étapes (e) à (f) pour rééquilibrer les billes d'hydrogel ;
(h) introduction de liquide pour remettre en suspension les billes d'hydrogel et la biomasse de champignons filamenteux et fragmentation de la biomasse de champignons filamenteux par aération ou agitation ;
(i) récolte de la biomasse de champignons filamenteux et des billes d'hydrogel via l'orifice de récolte (4) par rinçage ;
(j) facultativement, séchage du matériau récolté.

2. Procédé de production aseptique de champignons filamenteux dans un bioréacteur par fermentation à l'état pseudo-solide, comprenant les étapes de :
(a) introduction de billes d'hydrogel comprenant des champignons filamenteux viables dans une chambre (1) du bioréacteur en solution via un orifice d'inoculation (2) ou
production in situ de billes d'hydrogel à l'intérieur de la chambre (1) du bioréacteur par introduction d'au moins des champignons filamenteux, un agent de réticulation, et une solution de biopolymère dans la chambre (1) via un orifice d'inoculation (2), facultativement sachant que la solution de biopolymère comprend des nutriments, suivie par un lavage des billes produites ;
(b) si aucun nutriment n'est compris dans la solution de biopolymère à l'étape (a), équilibrage des billes d'hydrogel avec un milieu de croissance comprenant des nutriments ;
(c) distribution homogène des billes d'hydrogel sur au moins une plaque perforée (3) agencée horizontalement à l'intérieur de la chambre (1) par aération ou agitation ;
(d) élimination de tout ou pratiquement tout liquide de la chambre (1) via un orifice de récolte (4) ;
(e) fermentation des champignons filamenteux à l'intérieur du bioréacteur en l'absence de tout ou pratiquement tout liquide pour augmenter la biomasse de champignons filamenteux, sachant que les billes d'hydrogel sont le seul substrat de croissance et la seule matrice de croissance à l'intérieur du bioréacteur ;
(f) facultativement, au moins une introduction de milieu de croissance suivie par une répétition des étapes (d) à (e) pour rééquilibrer les billes d'hydrogel ;
(g) introduction de liquide pour remettre en suspension les billes d'hydrogel et la biomasse de champignons filamenteux et fragmentation de la biomasse de champignons filamenteux par aération ou agitation ;
(h) récolte de la biomasse de champignons filamenteux et des billes d'hydrogel via l'orifice de récolte (4) par rinçage ;
(i) facultativement, séchage du matériau récolté.

3. Procédé de production aseptique continue de champignons filamenteux dans un bioréacteur par fermentation à l'état pseudo-solide, comprenant les étapes de :
(a) introduction de billes d'hydrogel dans une chambre (1) du bioréacteur en solution via un orifice d'inoculation (2) ou
production in situ de billes d'hydrogel à l'intérieur de la chambre (1) du bioréacteur par introduction d'au moins un agent de réticulation et une solution de biopolymère dans la chambre (1) via un orifice d'inoculation (2), facultativement sachant que la solution de biopolymère comprend des nutriments, suivie par un lavage des billes produites ;
(b) si aucun nutriment n'est compris dans la solution de biopolymère à l'étape (a), équilibrage des billes d'hydrogel inoculées avec un milieu de croissance comprenant des nutriments ;
(c) inoculation de champignons filamenteux dans la chambre (1) via l'orifice d'inoculation (2) ;
(d) distribution homogène des billes d'hydrogel inoculées sur au moins une plaque perforée (3) agencée horizontalement à l'intérieur de la chambre (1) par aération ou agitation ;
(e) élimination de tout ou pratiquement tout liquide de la chambre (1) via un orifice de récolte (4) ;
(f) fermentation des champignons filamenteux à l'intérieur du bioréacteur en l'absence de tout ou pratiquement tout liquide pour augmenter la biomasse de champignons filamenteux, sachant que les billes d'hydrogel sont le seul substrat de croissance et la seule matrice de croissance à l'intérieur du bioréacteur ;
(g) facultativement, au moins une introduction de milieu de croissance suivie par une répétition des étapes (e) à (f) pour rééquilibrer les billes d'hydrogel ;
(h) introduction de liquide pour remettre en suspension les billes d'hydrogel et la biomasse de champignons filamenteux et fragmentation de la biomasse de champignons filamenteux par aération ou agitation ;
(i) drainage de liquide et de biomasse de champignons filamenteux en suspension via l'orifice de récolte (4), sachant que l'orifice de récolte comprend ou est recouvert par une plaque perforée supplémentaire, sachant que les ouvertures dans la plaque perforée supplémentaire ont un diamètre plus petit que les billes d'hydrogel de telle sorte que les billes d'hydrogel soient retenues dans la chambre (1) ;
(j) équilibrage des billes d'hydrogel avec un milieu de croissance ;
(k) distribution homogène des billes d'hydrogel et de toute biomasse de champignons filamenteux restante sur l'au moins une plaque perforée (3) agencée horizontalement à l'intérieur de la chambre (1) par aération ou agitation ;
(l) répétition continue des étapes (e) à (k) ;
(m) facultativement, séchage du matériau récolté.

4. Procédé de production aseptique continue de champignons filamenteux dans un bioréacteur par fermentation à l'état pseudo-solide, comprenant les étapes de :
(a) introduction de billes d'hydrogel comprenant des champignons filamenteux viables dans une chambre (1) du bioréacteur en solution via un orifice d'inoculation (2) ou
production in situ de billes d'hydrogel à l'intérieur de la chambre (1) du bioréacteur par introduction d'au moins des champignons filamenteux, un agent de réticulation, et une solution de biopolymère dans la chambre (1) via un orifice d'inoculation (2), facultativement sachant que la solution de biopolymère comprend des nutriments, suivie par un lavage des billes produites ;
(b) si aucun nutriment n'est compris dans la solution de biopolymère à l'étape (a), équilibrage des billes d'hydrogel avec un milieu de croissance comprenant des nutriments ;
(c) distribution homogène des billes d'hydrogel sur au moins une plaque perforée (3) agencée horizontalement à l'intérieur de la chambre (1) par aération ;
(d) élimination de tout ou pratiquement tout liquide de la chambre (1) via un orifice de récolte (4) ;
(e) fermentation des champignons filamenteux à l'intérieur du bioréacteur en l'absence de tout ou pratiquement tout liquide pour augmenter la biomasse de champignons filamenteux, sachant que les billes d'hydrogel sont le seul substrat de croissance et la seule matrice de croissance à l'intérieur du bioréacteur ;
(f) facultativement, au moins une introduction de milieu de croissance suivie par une répétition des étapes (d) à (e) pour rééquilibrer les billes d'hydrogel ;
(g) introduction de liquide pour remettre en suspension les billes d'hydrogel et la biomasse de champignons filamenteux et fragmentation de la biomasse de champignons filamenteux par aération ou agitation ;
(h) drainage de liquide et de biomasse de champignons filamenteux en suspension via l'orifice de récolte (4), sachant que l'orifice de récolte comprend ou est recouvert par une plaque perforée supplémentaire, sachant que les ouvertures dans la plaque perforée supplémentaire ont un diamètre plus petit que les billes d'hydrogel de telle sorte que les billes d'hydrogel soient retenues dans la chambre (1) ;
(i) équilibrage des billes d'hydrogel avec un milieu de croissance ;
(j) distribution homogène des billes d'hydrogel inoculées et de toute biomasse de champignons filamenteux restante sur l'au moins une plaque perforée (3) agencée horizontalement à l'intérieur de la chambre (1) par aération ou agitation ;
(k) répétition continue des étapes (e) à (j) ;
(l) facultativement, séchage du matériau récolté.

5. Le procédé de l'une quelconque des revendications 1 à 4, sachant que les billes d'hydrogel comprennent au moins un biopolymère et de l'eau ; de préférence sachant que l'au moins un biopolymère est sélectionné dans le groupe constitué par : l'alginate, le carraghénane, et la gomme gellane et/ou de préférence sachant que la teneur en eau des billes d'hydrogel est d'au moins 50 %, de préférence d'au moins 80 %, de manière plus préférentielle d'au moins 95 %.

6. Le procédé de la revendication 5, sachant que les billes d'hydrogel comprennent en outre un composé de charge de remplissage, de préférence sachant que le composé de charge de remplissage est sélectionné dans le groupe constitué par : le talc, l'amidon de maïs, la farine de maïs, la bentonite, la kaolinite, la poudre de silice de quartz, la tourbe, la gomme arabique.

7. Le procédé de l'une quelconque des revendications 1 à 6, sachant que les billes d'hydrogel ont une taille de 0,1 mm à 6 mm, de préférence de 0,5 mm à 5 mm, de manière la plus préférentielle de 2 mm à 4 mm.

8. Le procédé de l'une quelconque des revendications 1 à 7, sachant que les billes d'hydrogel forment une couche d'une hauteur de 1 cm à 150 cm, de préférence de 2 cm à 50 cm, de manière la plus préférentielle de 5 cm à 20 cm.

9. Le procédé de l'une quelconque des revendications 1 à 8, sachant que les billes d'hydrogel comprennent au moins une source de carbone, de préférence sachant que l'au moins une source de carbone est sélectionnée dans le groupe constitué par : un sucre, et un amidon ; de préférence sachant que le sucre est sélectionné dans le groupe constitué par : des monosaccharides et des disaccharides, et sachant que l'amidon est sélectionné dans le groupe constitué par : des oligosaccharides et des polysaccharides.

10. Le procédé de l'une quelconque des revendications 1, 3, et 5 à 9, sachant que l'inoculation avec des champignons filamenteux est une inoculation avec des spores.

11. Le procédé de l'une quelconque des revendications 1 à 10, sachant que l'équilibrage a lieu pendant 1 à 28 heures, de préférence 18 à 28 heures, de manière la plus préférentielle 24 heures.

12. Le procédé de l'une quelconque des revendications 1 à 11, sachant que pendant la fermentation, l'humidité à l'intérieur de la chambre (1) est régulée par aération via un aérateur à grosses bulles (5), de préférence sachant que l'humidité à l'intérieur de la chambre (1) est maintenue à entre 25 % et 100 %, de préférence à entre 70 % et 100 %.

13. Le procédé de l'une quelconque des revendications 1 à 12, sachant que la fermentation a lieu pendant 6 heures à 120 jours, de préférence pendant 1 à 90 jours.

14. Le procédé de l'une quelconque des revendications 1 à 13, sachant que les champignons filamenteux sont sélectionnés dans le groupe constitué par les Zygomycota, Ascomycota, Basidiomycota and Glomeromycota, de préférence dans la liste constituée par : Ampelomyces, Aspergillus, Beauveria, Fusarium, Isaria, Lecanicillium, Metarhizium, Penicillium, Phoma, Purpureocillium, Serendipita, Trichoderma, Verticillium et Mycorrhiza.

15. Le procédé de la revendication 1 ou 2, sachant que les champignons filamenteux sont des ectomycorhizes ou des mycorhizes arbusculaires (AMF).
